# EUROPÄISCHE PATENTSCHRIFT

(11) **EP 0 963 486 B1**
(45) Veröffentlichungstag und Bekanntmachung des Hinweises auf die Patenterteilung: **02.05.2003**
(21) Anmeldenummer: 98909444.6
(22) Anmeldetag: 10.02.1998
(51) Int. Cl.: D21H 21/22, D06M 13/224, D06M 13/342, D06M 13/388, D21H 17/07, D21H 17/14

(54) **FEUCHTIGKEITSREGULATOR ENTHALTENDE ZUSAMMENSETZUNG FÜR TISSUEPRODUKTE**
COMPOSITION CONTAINING A HUMIDITY REGULATOR, FOR TISSUE PRODUCTS
COMPOSITION CONTENANT UN REGULATEUR D'HUMIDITE DESTINEE AUX PRODUITS EN OUATE DE CELLULOSE

(30) Priorität: 19.03.1997 DE 19711452
(43) Veröffentlichungstag der Anmeldung: 15.12.1999
(73) Patentinhaber: SCA Hygiene Products GmbH, 68305 Mannheim (DE)
(72) Erfinder: NEUNHOEFFER, Hans, D-64367 Mühltal (DE); HILL, Walter, D-68623 Lampertheim (DE); EICHHORN, Stephan, D-64579 Gernsheim (DE); VON PALESKE, Peter, D-64342 Seeheim (DE)
(74) Vertreter: Sieckmann, Ralf, Dr.
(86) Internationale Anmeldenummer: EP9800767
(87) Internationale Veröffentlichungsnummer: WO98041687

(56) Entgegenhaltungen:
- EP-A- 0 186 208
- EP-A- 0 688 901
- DE-A- 2 622 571
- DE-A- 3 237 574
- DE-A- 4 334 367

## Beschreibung

Die vorliegende Erfindung betrifft wenigstens einen Feuchtigkeitsregulator enthaltende Zusammensetzung für Tissueprodukte, ein Verfahren zur Herstellung dieser Produkte, die Verwendung der Zusammensetzung für die Behandlung von Tissueprodukten sowie ein Tissueprodukt.

Die vorliegende Erfindung betrifft eine sowohl pflegende als auch weichheitsverbessernde Zusammensetzung zur Behandlung von Tissueprodukten, die beispielsweise bei Tissuepapieren wie Taschentüchern,

Kosmetiktüchern, Toilettenpapier, Küchentüchern und ähnlichem eingesetzt werden kann.

Papiergewebe, -blätter oder Vliese, im Hygienepapierbereich bei niedrigen Flächengewichten auch Tissue oder Tissuepapier bzw. -gewebe genannt, sind als Gebrauchsgüter aus der modernen Gesellschaft nicht mehr wegzudenken. Tissueprodukte, wie beispielsweise Taschentücher, Kosmetiktücher, Abschminktücher, Handtücher und Toilettenpapiere haben wesentlichen Anteil am Handelsvolumen, wobei ein Trend zu pflegenden Produkten geht. Bei Tissueprodukten mit speziellem Pflegecharakter rückt neben dem Pflegeaspekt noch zusätzlich der Aspekt extremer Weichheit der Tücher in den Mittelpunkt des Verbraucherinteresses. Im Hinblick auf pflegende Eigenschaften sind neben der erhöhten Weichheit der Produkte und einer bereits damit verbundenen passiven Pflege vor allen Dingen chemische Substanzen gemeint, die als Bestandteile einer Zusammensetzung auf das Tissue aufgebracht werden können. Unter dem Begriff "aktive Pflege" soll im folgenden verstanden werden, daß auf das Tissue eine Zusammensetzung aufgebracht wird, die beim Gebrauch dieses Tissues auf die Haut übergeht. Somit soll die Haut einerseits durch die Verwendung eines weichen Tuchs nicht noch zusätzlich gereizt werden und andererseits, bei einer entsprechenden ausreichenden Auftragsmenge, gepflegt und eine Hautrötung weitgehend verhindert oder zumindest gelindert wird. Als pflegende Bestandteile der Zusammensetzung sollen solche Substanzen verstanden werden, die auf der Haut besänftigend, entzündungshindernd oder -hemmend, feuchtigkeitsspendend und so weiter wirken, wie sie aus der Hautkosmetik als Bestandteile von Hautlotionen, Hautcremes, Hautsalben, Shampoos und so weiter bekannt sind.

Unter "passive Pflege" ist die Sanftheit eines Tissueproduktes, bedingt durch extreme Weichheit, zu verstehen, d. h. eine hohe Oberflächenglätte/Sanftheit in Verbindung mit hoher Knüllweichheit. Diese Weichheit kann über

Zusammensetzung und morphologischen Aufbau des Tissueprodukts, durch mechanische Maßnahmen wie Glätten oder Aufrauhen sowie durch Zusatz geeigneter chemischer Hilfsstoffe weiter verbessert werden.

Generell versteht man unter "Weichheit" bei Tissueprodukten das subjektive taktile Erlebnis des Verbrauchers, wenn dieser das Erzeugnis in die Hand nimmt und es über die Haut streicht bzw. es in der Hand zerknüllt. Wegen näherer Einzelheiten betreffend die Weichheit bzw. betreffend die Herstellung der Tissueprodukte verweisen wir auf die ältere Patentanmeldung WO96/08 601.

Der Einsatz von Chemikalien zur Erhöhung der Weichheit von Tissueprodukten ist ebenfalls bekannt. Meist handelt es sich bei diesen Chemikalien um kationische Tenside, wie sie in der deutschen Offenlegungsschrift 43 34 367 oder EP-A 0 688 901 beschrieben sind oder um ein Glukoseglutamat, wie man es im US-Patent 4 882 221 findet. Eine weitere Möglichkeit, weiche Tissuepapiere zu erhalten, ist die Verwendung von nichtionischen Tensiden, wie sie in der europäischen Patentanmeldung 347 177 beschrieben sind bzw. die Verwendung von Polysiloxanen, die unter anderem in den europäischen Patentanmeldungen 347 153 und 347 154 näher erörtert sind.

Weiter ist aus der DE-C 34 47 499 ein nichttrocknendes Reinigungstuch bekannt, bei dem auf einem Trägermaterial eine Emulsion aufgebracht ist, die mindestens aus einem Feuchtigkeitsregulator, vorzugsweise Polyethylenglykol und mindestens einem weiteren flüssigen Stoff besteht.

Darüber hinaus sind aus der WO96/08 601 bereits polysiloxanhaltige Zusammensetzungen für Tissuepapierprodukte bekannt, welche 25 - 95 Gew.-% wenigstens einer Polyhydroxyverbindung, 5 - 75 Gew.-% Polysiloxan sowie, bezogen auf 100 Gewichtsteile dieser Mischung, 0 - 35 Gew.-% Wasser enthalten. In dieser Anmeldung konnte gezeigt werden, daß die vorgenannte Kombination einen synergistischen Effekt auf die Weichheit hat. Der vorgenannte Stand der Technik hatte sich stets zuvor die Aufgabe gestellt, Tissueerzeugnisse mit erhöhter Weichheit bereitzustellen, bei denen die mechanischen Eigenschaften nicht negativ beeinflußt werden.

**US-A-4,786,367** betrifft ein Cellulosefasernetz, welches 0,1 - 2 Gew.-%, bezogen auf das Fasernetz, eines Lauroamphoglycinats enthält. Diese Druckschrift enthält allerdings keine Angaben, die darauf hinweisen, daß dieses Fasernetz zusätzlich noch Feuchtigkeitsregulatoren enthält, noch legt sie dies nahe.

Die **deutsche Offenlegungsschrift 32 37 574** betrifft die Verwendung spezieller Aminoxide, wie sie u.a. durch die allgemeine Formel (I) nachfolgend definiert ist, als Hilfsmittel bei der Herstellung von Zellstoff, Papier oder speziellen Hygieneprodukten zur Herabsetzung der mechanischen Festigkeit und zur Verbesserung der Benetzbarkeit bzw. des Wasseraufnahmevermögens. Diese Aminoxide werden üblicherweise in Mengen von 0,02- 2,0 %, vorzugsweise 0,01 - 1 %, bezogen auf das Trockengewicht der Cellulose, zugesetzt. Wiederum wird durch diese Druckschrift weder vorbeschrieben noch nahegelegt, derartigen Zusammensetzungen für Tissueprodukte oder Tissueprodukten selbst, Feuchtigkeitsregulatoren zuzusetzen.

Die **EP-A-0 347 176** betrifft ein Tissuepapier mit einem Flächengewicht von 10 - 65 Gramm pro m² und einer Dichte von nicht mehr als 0,6 Gramm pro cm³, wobei dieses Papier neben Cellulosefasem wenigstens 0,01 Gew.-% eines nicht-kationischen Tensids; bezogen auf das Trockenfasergewichts des Papiers enthält, wobei besagtes nicht-kationisches Tensid auf das feuchte Tisssuenetz aufgebracht worden ist. Unter dem vorgenannten nicht-kationischen Tensid versteht man u.a. ein wasserlösliches Aminoxid mit einer Alkylgruppe mit 10 - 15 Kohlenstoffatomen und 2 (Hydroxy)Alkylgruppen mit 1 - 3 Kohlenstoffatomen. Wiederum ist dieser Druckschrift nicht zu entnehmen, einer entsprechenden, dieses entsprechende Tensid enthaltenden Zusammensetzung weiterhin Feuchtigkeitsregulatoren beizufügen und diese Mischung auf Tissueprodukten einzusetzen, noch wird dies nahegelegt.

Die **EP-A-0 347 177** definiert das in der EP-A 0 347 176 genannte Erzeugnis, als das entsprechende Herstellverfahren. Insofern wird auch hierdurch der derzeit beanspruchte Gegenstand weder vorbeschrieben noch nahegelegt.

Die **EP-A-0 688 901** betrifft ein Tissuepapiererzeugnis, zu dem 3 - 35 Trocken-Gew.-% einer wässrigen, weichheitsverleihenden Zusammensetzung hinzugefügt worden sind, wobei besagte, die Weichheit verleihende Zusammensetzung aus 20 - 98 Gew.-% Glycerin und 0,2 - 5 Gew.-% einer quaternären Ammoniumverbindung enthält, worunter man, üblicherweise ein kationisches und kein nicht-ionisches oder ampho-ionisches Tensid versteht. Insofern wird durch diese Druckschrift weder vorbeschrieben noch nahegelegt, spezielle Mengen eines speziellen nicht-ionischen Tensids einzusetzen. Eine Substitution des kationischen Tensids gegen ein nicht-ionisches Tensid wird auch nicht nahegelegt, da beide Stoffklassen aufgrund des unterschiedlichen Anwendungsspektrums üblicherweise nicht miteinander vergleichbar sind.

Die **DE-C-34 47 499** betrifft ein nichttrocknendes Reinigungstuch, bestehend aus einem flächigen Trägermaterial, auf das eine Emulsion aufgebracht ist, die Wasser und einen Feuchtigkeitsregulator enthält, wobei auf das Trägermaterial zwingend feinteilige, anorganische und / oder organische dispergierbare Feststoffe aufgebracht worden sind. Auch dieser Druckschrift ist es nicht zu entnehmen, einer entsprechenden Zusammensetzung für Tissueprodukten oder Tissueprodukten selbst spezielle Mengen eines speziellen nicht-ionischen Tensids, ohne die entsprechenden feinteiligen, anorganischen und / oder organischen dispergierbaren Feststoffen zuzusetzen.

Die **EP-A-0 282 289** betrifft eine die Haut behandelnde Zusammensetzung enthaltend ein bis 35 Gew.-% eines Salzes eines Citronensäuremonoalkylesters. Als Träger dieser Zusammensetzung wird ein Paraffinöl oder pflanzliches Öl eingesetzt. Hiermit wird weder vorbeschrieben noch nahegelegt, als weitere Komponente einen hydrophilen Feuchtigkeitsregulator, insbesondere auf Basis von Polyolen einzusetzen und dies speziell in Hinblick auf Tissueprodukte.

Die deutsche **Auslegeschrift 26 22 571** betrifft ein mit reinigenden, rückfettenden sowie bakteriostatischen Stoffen imprägniertes Feucht-Toilettenpapier, wobei der Trägerstoff mit einer Lösung folgender Zusammensetzung getränkt ist: 0,2 - 2,0 Gew.-% Fettsäureamidoalkylbetain, 0,2 - 2,0 Gew.-% ethoxylierte Glycerinpartialester gesättigter Fettsäuren, 0 - 20 Gew.-% aliphatische Alkohole mit 2 - 4 C-Atomen, 0,5 - 5 Gew.-% organische Säuren, 0 - 0,5 Gew.-% Parfumöl und Wasser als Rest. Bedingt durch den Anteil der gegebenenfalls zuzufügenden Säuren wird ein pH-Wert von 5 - 6 erreicht. Worum es sich im einzelnen bei der Komponente aliphatische Alkohole handeln soll, ist dort ebenfalls definiert, es soll sich nämlich um desinfizierende und konservierende Komponenten handeln, worunter man üblicherweise Niederalkylmonoalkohole versteht, insbesondere die in der Druckschrift genannten Vertreter Ethanol und Isopropanol. Hierdurch ist weder vorbeschrieben noch nahegelegt, dieser Zusammensetzung Feuchtigkeitsregulatoren zuzusetzen.

Die **DE-U-85 12 083** betrifft ein Feuchttoilettenpapier, welches dadurch gekennzeichnet ist, daß es aus einem mit Glykol oder Glykolester getränktem

Papier besteht. Diesem Dokument ist weder zu entnehmen, noch wird durch dieses nahegelegt, dem Feuchttoilettenpapier weitere Komponenten zuzufügen, welche sowohl eine weichheitsfördernde als auch eine pflegende Wirkung für den Benutzer haben.

Die **AT-358 741** betrifft ein mit Reinigungsemollienz imprägniertes Hautreinigungsvlies, bei dem das Vlies eine Wischoberfläche und eine Wischzone niedriger Dichte aufweist, wobei die Wischoberfläche eine Grenze der Wischzone darstellt, die Wischzone einen mittleren Hohlraumindex von mindestens 68 und einen darüber hinaus durch die aufgebrachte Zusammensetzung auch noch eine aktive Pflegewirkung hat.

Gegenstand der vorliegenden Erfindung ist somit eine wenigstens einen Feuchtigkeitsregulator enthaltende Zusammensetzung für Tissueprodukte bereitzustellen, die sowohl eine weichheitsfördernde als auch eine pflegende Wirkung für den Benutzer hat.

Die vorliegende Erfindung betrifft somit einen wenigstens einen Feuchtigkeitsregulator enthaltende Zusammensetzung für Tissueprodukte, die dadurch gekennzeichnet ist, daß sie weiterhin 1 bis 25 Gew.-%, vorzugsweise 2 bis 20 Gew.-%, bezogen auf die Gesamtzusammensetzung, wenigstens eines speziellen nichtionischen Tensids ausgewählt aus wenigstens einem Aminoxid und/oder Hydroxysäureestern, ausgenommen Myristyllactat und Natriumsalze von Fettalkohollactaten, und/oder wenigstens eines Amphotensids enthält und wobei der Feuchtigkeitsregulator in einer Menge von 40 bis 90 Gew.-%, bezogen auf die Gesamtzusammensetzung, enthalten ist.

Weiterer Gegenstand der vorliegenden Erfindung ist ein Verfahren zur Herstellung von Tissueprodukten, bei denen eine derartige Zusammensetzung auf das Faservlies oder die Tissuebahn entweder innerhalb der Siebpartie, der Pressenpartie, der TAD-Partie, am Yankee-Zylinder und/oder der Trockenpartie, d. h. bei einer Faserstoffdichte von 20 bis 97 %, bezogen auf das Trockenfasergewicht der Bahn, in einer Menge von 0,1 bis 50 Gew.-% kontinuierlich oder diskontinuierlich auf oder in der Bahn appliziert und die Bahn gegebenenfalls nach der Applikation einer Nachglättung unterzogen wird.

Alternativ liegt der vorliegenden Erfindung die Aufgabe zugrunde, ein Verfahren zur Herstellung von Tissueprodukten bereitzustellen, bei dem die vorgenannte Applikation nach der Trockenpartie an der Wattemaschine, der Doubliermaschine und / oder in dem Verarbeitungsautomat in vorgenannter Weise in einer Menge von 0,1 bis 40 Gew.-%, vorzugsweise 1 bis 20 Gew.-%, appliziert wird und gegebenenfalls einer Nachglättung unterzogen wird.

Weiterer Gegenstand der vorliegenden Erfindung ist ein Tissueprodukt enthaltend eine Feuchtigkeitsregulator enthaltende Zusammensetzung, das dadurch gekennzeichnet ist, daß es weiterhin 1 bis 25 Gew.-%, vorzugsweise 2 bis 20 Gew.-%, bezogen auf die Gesamtzusammensetzung, wenigstens eines speziellen, nichtionischen Tensids, ausgewählt aus wenigstens einem Aminoxid und/oder Hydroxysäureestern, wobei Myristyllactat und Natriumsalze von Fettalkohollactaten ausgenommen sind, und/oder wenigstens eines Amphotensids enthält und wobei der Feuchtigkeitsregulator in einer Menge von 40 bis 90 Gew.-%, bezogen auf die Gesamtzusammensetzung, enthalten ist.

Gegenstand der vorliegenden Erfindung ist schließlich die Verwendung der vorgenannten Zusammensetzung, insbesondere in Lotionsform, für die Behandlung von Tissueprodukten, insbesondere Taschentüchern, Kosmetiktüchern, Abschminktüchern, Toilettenpapier und Küchentüchern.

Nach einer bevorzugten Ausführungsform der erfindungsgemäßen Zusammensetzung ist das vorgenannte, nichtionische Tensid ein Aminoxid mit der allgemeinen Formel 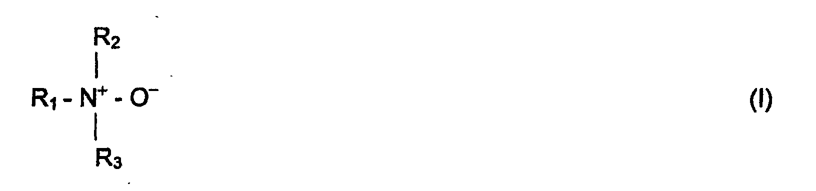 in der R₁, R₂ und R₃ unabhängig voneinander für einen gegebenenfalls substituierten aliphatischen, linearen oder verzweigten Alkylrest mit 1 bis 25 Kohlenstoffatomen, vorzugsweise 1 bis 17 Kohlenstoffatomen, für einen gegebenenfalls substituierten, cyclischen Alkylrest mit 3 bis 25 C-Atomen, vorzugsweise 3 bis 17 C-Atomen, für einen gegebenenfalls substituierten aliphatischen, linearen oder verzweigten Amidoalkylrest mit 3 bis 22 C-Atomen, vorzugsweise 7 bis 17 C-Atomen im Alkylteil oder für einen gegebenenfalls substituierten cyclischen Amidoalkylrest mit 3 bis 10 C-Atomen, vorzugsweise 5 bis 8 C-Atomen im Alkylteil steht.

Bei diesen Aminoxiden ist es bevorzugt, daß bei der vorgenannten Formel R₁ und R₂ eine Alkylgruppe mit 1 bis 4 C-Atomen, vorzugsweise Methyl sind und R₃ eine Amidoalkylgruppe der allgemeinen Formel (CH₂)ₐN(H)C(O)R₄ (II) ist, bei der a 1 bis 5 ist und R₄ ein aus natürlichen Ölen oder Fetten stammender Fettsäurerest mit 7 bis 25 C-Atomen, vorzugsweise 7 bis 17 C-Atomen ist.

Nach einer weiteren bevorzugten Ausführungsform enthalten die erfindungsgemäßen Zusammensetzungen einen Hydroxysäureester, wobei es sich beispielsweise um einen Glucosesäureester, einen Milchsäureester, einen Mandelsäureester, einen Äpfelsäureester, einen Weinsäureester, einen Citronensäureester oder einen Rizinolsäureester handeln kann, der wenigstens einen aus natürlichen Ölen oder Fetten stammenden Fettsäurerest mit 1 bis 25 C-Atomen, vorzugsweise 7 bis 17 C-Atomen aufweist, der gegebenenfalls weiter wenigstens eine polare Gruppe wie beispielsweise Hydroxi aufweist. In diesem Zusammenhang sind entsprechende, wenigstens einen Fettsäurerest enthaltende Citronensäureester und deren Salze besonders bevorzugt.

Nach einer weiteren bevorzugten Ausführungsform weist die erfindungsgemäße Zusammensetzung ein Amphotensid in Form eines Ampholyts oder eines Betains auf.

Sofern der in der erfindungsgemäßen Zusammensetzung enthaltene Bestandteil ein Betain ist, so sollte dieses aus natürlichen oder synthetischen Quellen abgeleitet sein und ausgewählt sein aus Alkylbetainen der allgemeinen Formel R₅(CH₃)N⁺(CH₃)CH₂COO⁻(III), wobei R₅ eine gegebenenfalls substituierte lineare oder verzweigte Alkylgruppe mit 1 bis 25 C-Atomen, vorzugsweise 7 bis 17 C-Atomen aufweist, Alkylamidobetainen der allgemeinen Formel, R₆ (CO)NH(CH₂)ₒ(CH₃)N⁺ (CH₃)CH₂COO⁻ (IV), wobei R₆ eine gegebenenfalls substituierte lineare oder verzweigte Alkylgruppe mit 1 bis 25 C-Atomen, vorzugsweise 7 bis 17 C-Atomen ist und o gleich 1 bis 5 ist, Sulfobetainen der allgemeinen Formel R₇(CH₃)N⁺(CH₃)-X-SO₃₋ (VI), wobei R₇ eine gegebenenfalls substituierte Alkylgruppe mit 1 bis 25 C-Atomen, vorzugsweise 7 bis 17 C-Atomen und X eine gegebenenfalls substituierte Alkylengruppe mit 1 bis 5 Methyleneinheiten ist, Alkylpolyamphopolycarboxyglycinate der allgemeinen Formel 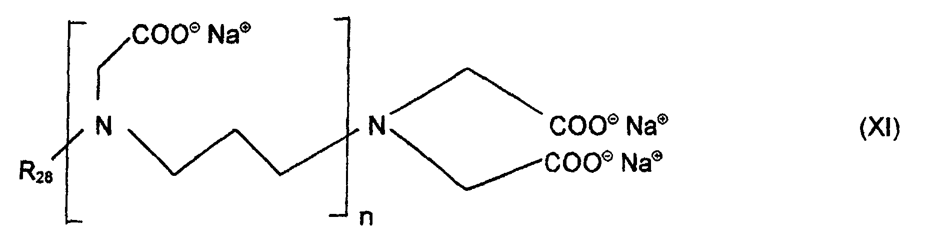 wobei R₂₈ eine C₇ - C₁₇-Fettsäure und n gleich 2 oder 3 ist oder Glycinester der allgemeinen Formel 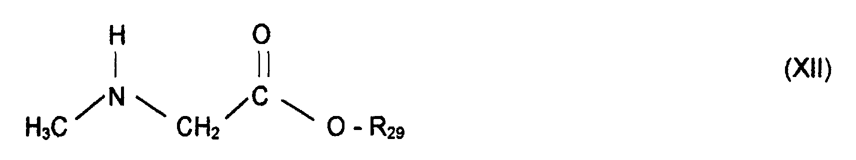 wobei R₂₉ eine C₇ - C₁₇-Alkylgruppe, vorzugsweise C₁₂H₂₅ ist.

Bei der erfindungsgemäßen Zusammensetzung ist es weiterhin bevorzugt, daß hierin der Feuchtigkeitsregulator in einer Menge von 40 bis 90 Gew.-%, vorzugsweise 50 bis 80 Gew.-%, bezogen auf die Gesamtzusammensetzung, enthalten ist und hierbei insbesondere ausgewählt ist aus Polyolen. Unter Polyolen im Sinne der vorgenannten Art versteht man insbesondere Glycerin, Polyalkylenglykol, 1,3-Butylenglykol (Butylenglykol), Propylenglykol und Zuckeralkohole, wobei insbesondere Glycerin, Butylenglykol und/oder Propylenglykol besonders bevorzugt sind.

Nach einer weiteren bevorzugten Ausführungsform enthalten die erfindungsgemäßen Zusammensetzungen 0,01 bis 50 Gew.-%, vorzugsweise 0,1 bis 40 Gew.-% wenigstens eines natürlichen, naturidentischen oder synthetischen Hautpflegemittels. Zu nennen sind hier Hautpflegemittel auf Basis von Vitaminen oder Pflanzenextraken, wie beispielsweise Extrakte von Roßkastaniensamen, Birke, Arnika, Kamille oder Bisabolol bzw. Azulen selbst, Johanniskraut, Gurke, Aloe Vera, Hopfen, Allantoin oder Hamamelis und Linde, welche zum Teil auch wegen ihrer adstringierenden und heilungsfördernden Wirkung bekannt sind. Provitamin B5 = D-Panthenol ist besonders gut geeignet, da er gleichzeitig als Feuchthaltemittel dient und so zum Teil das Glycerin/Propylenglykol ersetzt. Im Falle von Bisabolol und Azulen zeigt bereits ein Zusatz von 0,5 bis 1 % zur Zusammensetzung einen entsprechenden Effekt. Andere Wirkstoffe, die in die Zusammensetzung eingegeben werden können, sind Glycyrrhethinsäure, der Wirkstoff aus der Wurzel des Süßholzstrauches, der bakteriostatisch und entzündungshemmend wirkt, sowie deren Salze und Phytosterol (auch ethoxyliert) Generol® (Henkel KGaA), hergestellt aus Sojaöl, das ebenfalls entzündungshemmend wirkt (R. Wachter, B. Salka und A. Magnet, Parfümerie und Kosmetik 75 (1994) 755). Die Zusammensetzung kann 1 bis 5 Gew.-% dieser Wirkstoffe enthalten. Als weitere Wirkstoffe sind hier beispielsweise Sorbitanfettsäureester oder oxethylierte homologe Verbindungen des Glycerins, Ester von oxethylierten Fettalkoholen, Fettalkoholalkanolamide, oxethylierte Fettalkohole, oxethylierte Wollfettalkohole, Glycerinmonostearat, Stearinsäure, Cetylstearylalkohol, Vaseline und Lanolin zu nennen. Neben Lanolin selbst können auch Lanolinderivate eingesetzt werden, wie beispielsweise Lanolinalkohol oder Wollwachsalkohole, die unter dem Namen Amerchol von der Union Carbide Inc. in Verbindung mit Mineralölen vertrieben werden. Weitere Lanolinderivate sind die acetylierten Lanoline sowie hydrophile Lanolinderivate, beispielsweise Lanolinpolyoxiethylen-Verbindungen. Als sinnvolle ergänzende Pflegebestandteile kann die Zusammensetzung weiterhin Jojobaöl, Avocadoöl, Teebaumöl und Lindenextrakt oder Reiskeimöl in der Zusammensetzung in einer Menge zwischen 1 und 40 Gew.-%, insbesondere aber 10 bis 20 Gew.-% enthalten.

Die als ein möglicher Bestandteil des Hautpflegemittels eingesetzte Polysiloxankomponente kann eine beliebige wasserlösliche und/oder wasserdispergierbare Verbindung, die bei Raumtemperatur (20 °C) flüssig, pastenförmig oder wachsartig vorliegt, sein. Die für die Zwecke der vorliegenden Erfindung eingesetzte Polysiloxankomponente schließt polymere, oligomere, copolymere und andere multiple, monomere Siloxane ein. Im folgenden soll man unter dem Begriff Polysiloxan jegliches polymeres, oligomeres oder anderes mehrfachmonomeres Siloxanmaterial verstehen. Weiterhin kann das Polysiloxanmaterial sowohl eine lineare Struktur, eine verzweigte Struktur oder eine cyclische Struktur aufweisen.

Nach einer bevorzugten Ausführungsform der Zusammensetzung weist die Polysiloxankomponente im Hautpflegemittel monomere Siloxaneinheiten der folgenden Struktur: 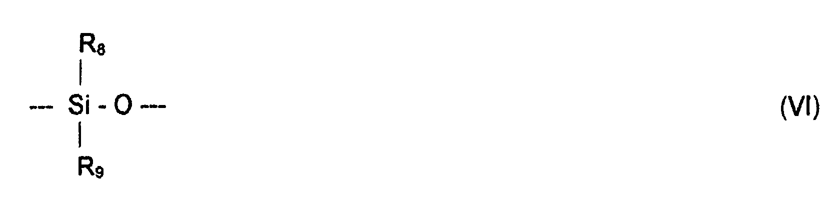 auf, wobei R₈ und R₉ für jede monomere Siloxaneinheit gleich oder verschieden sind und jeweils eine Alkyl-, Aryl-, Alkenyl-, Alkylaryl-, Arylalkyl-, Cycloalkyl-, halogenierte Kohlenwasserstoff- oder andere Gruppe ist. Jede dieser Gruppen kann substituiert oder unsubstituiert sein. R₈ und R₉-Gruppen von jeder speziellen monomeren Einheit können sich von den entsprechenden funktionellen Gruppen der nächsten anhängenden monomeren Einheit unterscheiden. Weiterhin können diese Gruppen sowohl geradkettig wie auch verzweigt sein oder eine cyclische Struktur aufweisen. Die Gruppen R₈ und R₉ können weiterhin und unabhängig voneinander andere Silikongruppen sein, aber sind nicht auf Siloxane, Polysiloxane und Polysilane beschränkt. Die Gruppen R₈ und R₉ können weiterhin eine große Anzahl von organischen funktionellen Gruppen enthalten, beispielsweise Alkohol, Carbonsäure und aminofunktionelle Gruppen.

Der Substitutionsgrad und die Substitutionsart bewirken den relativen Grad der Weichheit, des seidigen Griffs und der Hydrophilität, die der Tissuepapierstruktur verliehen wird. Im allgemeinen steigt der Grad der Weichheit und des seidigen Griffs, der unter anderem von dem Polysiloxan bewirkt wird, sofern die Hydrophilität der substituierten Polysiloxankomponente abnimmt. Aminofunktionelle Polysiloxane und Polyetherpolysiloxane sind als Polysiloxankomponente im erfindungsgemäßen Behandlungsmittel besonders bevorzugt.

Bevorzugte hautfreundliche Polysiloxane in der erfindungsgemäßen Zusammensetzung schließen lineare Organo-Polysiloxanverbindungen der folgenden allgemeinen Formel ein, 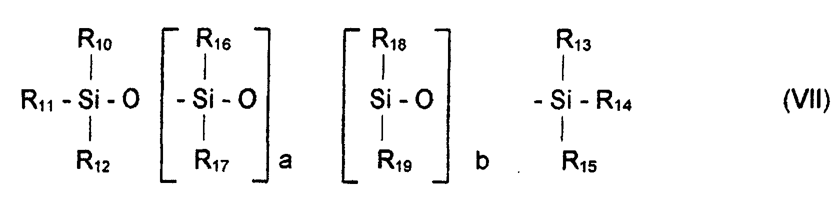 wobei jeweils die R₁₀ bis R₁₈-Gruppen unabhängig voneinander C₁ bis C₁₀ unsubstituierte Alkyl- oder Arylgruppen sind und R₁₉ ein beliebig substituiertes C₁ bis C₁₀-Alkyl- oder Arylradikal ist. Vorzugsweise ist jede R₁₀ bis R₁₈-Gruppe unabhängig voneinander eine C₁ bis C₁₀ unsubstituierte Alkylgruppe. Dem Fachmann auf diesem Gebiete ist es bekannt, daß es keinen großen Unterschied ausmacht, ob beispielsweise R₁₈ oder R₁₉ die substituierte Gruppe ist. Vorzugsweise beträgt das Molverhältnis von b zu (a+b) zwischen 0 und 20 %, vorzugsweise zwischen 0 und 10 % und insbesondere zwischen 1 und 5 %.

Nach einer besonders bevorzugten Ausführungsform der erfindungsgemäßen Zusammensetzung sind im hautfreundlichen Polysiloxan R₁₀ bis R₁₈ Methylgruppen, und R₁₉ ist eine substituierte oder unsubstituierte Alkyl-, Aryl- oder Alkenylgruppe. Derartige Materialien werden im allgemeinen hier als Polydimethylsiloxane bezeichnet, die eine spezielle Funktionalität aufweisen, wie sie im vorliegenden Fall eingesetzt werden. Beispiele für derartige Polydimethylsiloxane können sein: Polydimethylsiloxane wie Dow Corning® 200 Fluid, Polydimethylcyclosiloxane wie Dow Corning® 344 und 345, Polydimethylsiloxan mit einer R₁₀-Alkylkohlenwasserstoffgruppe und ein Polydimethylsiloxan mit einem oder mehreren Amino-, Carboxyl-, Hydroxyl-, Ether-, Polyether-, Aldehyd-, Keton-, Amid-, Ester-, Thiol- und/oder anderen R₁₉ funktionellen Gruppen ein, eingeschlossen Alkyl- und Alkenylanaloge solcher funktioneller Gruppen. Beispielsweise kann eine aminofunktionelle Alkylgruppe wie R₁₉ ein aminofunktionelles oder ein aminoalkylfunktionelles Polydimethylsiloxan sein. Die beispielhafte Aufzählung dieser Polydimethylsiloxane bedeutet nicht, daß andere, nicht speziell hier genannte, hiervon ausgeschlossen sind.

Die Viskosität der als hautfreundliche Komponente in der erfindungsgemäßen Zusammensetzung eingesetzten Polysiloxane kann über einen weiten Bereich variieren, solange das Polysiloxan fluid bleibt und für die Verwendung in dem erfindungsgemäßen Behandlungsmittel zur Applikation auf dem Tissuepapier verflüssigt werden kann. Hierunter versteht man beispielsweise Viskositäten von 25 x 10⁻⁶ m²/s bis 20.000.000 x 10⁻⁶ m²/s oder sogar höher. Bevorzugt sind hier Viskositäten von 15.000 x 10⁻⁶ m²/s bis 3.400.000 x 10⁻⁶ m²/s. Hochviskose Polysiloxane, die selbst nicht fließfähig sind, können als Bestandteil der erfindungsgemäßen Zusammensetzung auf Tissuepapier in wirksamer Weise aufgebracht werden, indem man beispielsweise die Polysiloxankomponente erfindungsgemäß in Polyol oder Propylenglykol bzw. Glycerin bzw. Wasser oder in deren Mischung gelöst zusammen mit einem Tensid emulgiert oder das Polysiloxan, sofern es nicht in Propylenglykol bzw. Glycerin bzw. Wasser lösbar ist, mittels eines Lösemittels, wie beispielsweise Hexan, in Lösung bringt. Spezielle Methoden, um die Polysiloxankomponente auf Tissueprodukte aufzubringen, werden im folgenden diskutiert.

Die vorgenannten hautfreundlichen Polysiloxankomponenten sind beispielsweise in US-A-2826551, US-A-3964550, US-A-4364837, US-A-4395454, US-A-4950545, US-A-4921895 und britische Patentschrift 849433 beschrieben. Weiterhin enthält die Monographie "Silicon Compounds", Seite 181 - 217, herausgegeben von Petrarch Systems, 1984, eine ausführliche Auflistung und Beschreibung derartiger Polysiloxane.

Gemäß einer weiteren bevorzugten Ausführungsform können als hautfreundliche Polysiloxankomponente in den erfindungsgemäßen Zusammensetzungen Polyethersiloxane der allgemeinen mittleren Formel eingesetzt werden, 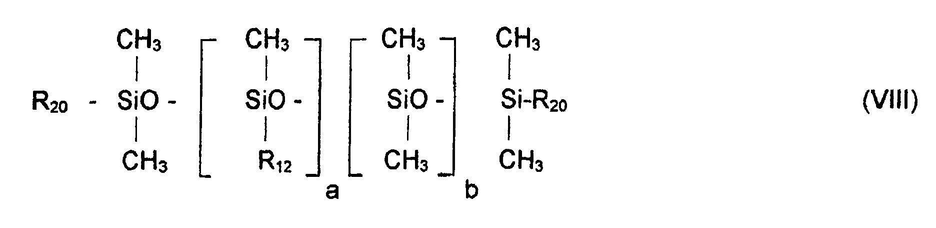 in denen R₂₀ in dem Molekül gleich oder verschieden sind und eine Alkylgruppe mit 1 bis 12 Kohlenstoffatomen oder eine Polyethergruppe- (CₙH₂ₙO)ₓ R₂₁, wobei R₂₁ Wasserstoff, Hydroxyl, Alkyl oder eine Acylgruppe ist und n einen Zahlenwert von 2 bis 2,7 aufweist und x einen numerischen Wert von 2 bis 200 besitzt, mit der Maßgabe, daß wenigstens eine der R₂₀-Gruppen im Durchschnittsmolekül eine Polyethergruppe ist; a einen numerischen Wert von 0 bis 98 hat, b einen numerischen Wert von 0 bis 98 hat und a+b 8 bis 98 ist. R₂₀ kann eine Alkylgruppe mit 1 bis 12 Kohlenstoffatomen oder eine Polyethergruppe sein. Allerdings muß die Bedingung erfüllt sein, daß wenigstens ein R₂₀ im durchschnittlichen Molekül eine Polyethergruppe ist. Vorzugsweise sind 2 bis 5 der R₂₀-Gruppen Polyethergruppen, und die verbleibenden R₂₀-Gruppen haben dann die Bedeutung einer Alkylgruppe, wobei die Methylgruppe besonders bevorzugt ist. Die Alkylgruppe kann aber auch bis zu 12 Kohlenstoffatomen aufweisen. Auf diese Weise ist es möglich, die Eigenschaften des Behandlungsmittels zu variieren und auf diese Weise die Handhabung auf Tissuepapierprodukten zu verbessern. Die Polyethergruppen entsprechen der Formel (CₙH₂ₙO)ₓR₂₁. Der Index n hat einen Zahlenwert von 2 bis 2,7. Im allgemeinen besteht die Ethergruppe aus einer Mehrzahl von Oxyethylenen und gegebenenfalls Oxypropylengruppen. Wenn der Index n 2 ist, besteht die Polyethergruppe ausschließlich aus Oxyethyleneinheiten. Sofern der Zahlenwert von n ansteigt, steigt ebenfalls der Anteil der Oxypropylengruppen an. Der numerische Wert von n = 2,7 bedeutet, daß 70 % der Polyethergruppen Oxypropylengruppen sind.

Der Index x bedeutet die Anzahl der Oxyalkyleneinheiten. Dieser Wert ist ein mittlerer Zahlenwert, da eine Mischung der Produkte von unterschiedlicher Kettenlänge üblicherweise bei der Synthese von Polyethern erhalten wird. Der Index x weist einen Zahlenwert von 2 bis 200 auf und liegt vorzugsweise bei 10 bis 50. Polyethergruppen mit einem mittleren Molekulargewicht von 600 bis 4.000 sind bevorzugt. Der Index a bedeutet die Anzahl der Methylsiloxaneinheiten, die von der R₁₂-Gruppe getragen werden. Der Index b entspricht der Anzahl der Dimethylsiloxaneinheiten. Während a und b einem Wert von 0 bis 98 annehmen können, muß die Bedingung erfüllt sein, daß die Summe von a+b einen Wert von 8 bis 98 besitzt. Wenn a = 0 ist, ist die Polyethergruppe oder -gruppen endständig verbunden. Die Siloxane mit positiven Werten für a werden durch die R₁₂-Seitenketten modifiziert. Siloxane, bei denen die R₂₀-Gruppen in der Seitenkette angeordnet sind, sind bevorzugt. Die R₂₁-Gruppe kann Wasserstoff, Hydroxyl, Alkyl oder auch Acyl sein. Vorzugsweise ist R₁₃ ein Wasserstoffatom. Sofern R₂₁ eine Alkylgruppe ist, so sind niedere Alkylgruppen mit 1 bis 4 Kohlenstoffatomen bevorzugt. Die Acetylgruppe ist die bevorzugte Acylgruppe.

Nach einer besonders bevorzugten Ausführungsform weist die hautfreundliche Polysiloxankomponente in der erfindungsgemäßen Zusammensetzung folgende Formel auf: 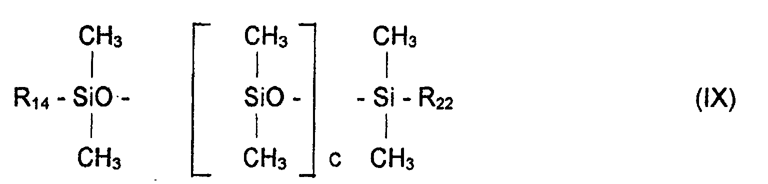 wobei R₂₂ eine Gruppe der Formel 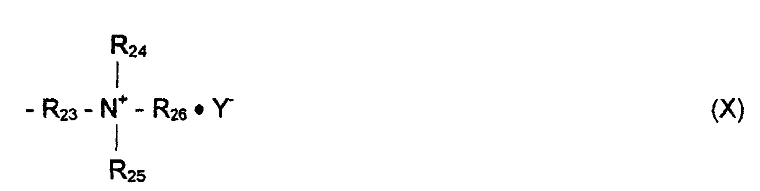 und in denen R₂₃ eine bi-valente Kohlenwasserstoffgruppe ist, deren Kohlenstoffkette durch ein Sauerstoffatom unterbrochen ist, R₂₄, R₂₅, R₂₆ gleich oder unterschiedlich sind und Alkylgruppen mit 1 bis 18 Kohlenstoffatomen darstellen, von denen eine der Gruppen R₂₄, R₂₅, R₂₆ eine -(CH₂)₃ NHCOR₁₉-Gruppe ist, bei denen R₁₉ eine Alkylgruppe mit 7 bis 17 Kohlenstoffatomen und Y⁻ ein einwertiges Anion und c einen Zahlenwert von 5 bis 100 aufweist. R₂₃ ist eine divalente Kohlenwasserstoffgruppe, beispielsweise die Gruppe der Formel -CH₂-C(OH)H-CH₂-O-(CH₂)₃-. Die R₂₄-, R₂₅-, R₂₆-Gruppen können gleich oder verschieden sein und sind Alkylgruppen mit 1 bis 18 Kohlenstoffatomen. Allerdings kann eine der vorgenannten Gruppen R₂₄, R₂₅, R₂₆ auch die Bedeutung einer (CH₂)₃ NHCOR₂₇-Gruppe haben.

Sofern R₂₄-, R₂₅-, R₂₆-Gruppen Alkylgruppen sind, so weisen diese 1 bis 18 Kohlenstoffatome auf. Besonders bevorzugt sind R₁₄-Gruppen, in denen zwei der vorgenannten R₂₄-, R₂₅-, R₂₆-Gruppen 1 bis 4 Kohlenstoffatome aufweisen und die dritte Gruppe bis zu 18 Kohlenstoffatome besitzt. Sofern eine der R₂₄-, R₂₅-, R₂₆-Gruppen eine (CH₂)₃ NHCOR₂₇-Gruppe ist, so ist die R₁₉-Gruppe eine Alkylgruppe mit 7 bis 17 Kohlenstoffatomen. Y⁻ ist ein einwertiges Anion, im allgemeinen eine Acetatgruppe. Y kann allerdings auch eine anorganische Gruppe wie beispielsweise Cl⁻ sein. Der Index "c" gibt die Anzahl der Dimethylsiloxyeinheiten im linearen Siloxan an und hat einen Zahlenwert von 5 bis 100 und vorzugsweise 10 bis 80. Besonders bevorzugt von den vorgenannten Siloxanen sind solche Polydimethylsiloxane sowie beispielsweise Polyether-, Alkyl-, sowie mit quaternären oder betainischen Gruppen, insbesondere Stickstoffgruppen modifizierte Polydimethylsiloxane.

Besonders bevorzugte Polysiloxane sind die unter der Bezeichnung Tegopren® von der Th. Goldschmidt AG vertriebenen organo-modifizierten Siloxane mit ausgeprägter Oberflächen- und Grenzflächen-Aktivität in wäßrigen und organischen Systemen, Dies sind Polyethersiloxane, wie sie in der Firmendruckschrift "Tegopren® Informativ", undatiert, der Th. Goldschmidt AG unter dem Handelsnamen Tegopren® 3012, Tegopren® 3020, Tegopren® 3021, Tegopren® 3022, Tegopren® 3070, Tegopren® 5830, Tegopren® 5840, Tegopren® 5842, Tegopren® 5843, Tegopren® 5847, Tegopren® 5851, Tegopren® 5852, Tegopren® 5863, Tegopren® 5873, Tegopren® 5878, Tegopren® 5884 sowie Tegopren® 7006 vertrieben werden und üblicherweise mittlere Trübungspunkte im Bereich von unter 25 °C bis 71 °C aufweisen sowie modifizierte Siloxane in Form von Tegopren-Silikon-Quats und -Betainen, wie sie unter den Bezeichnungen Tegopren® 6920, Tegopren® 6922 und Tegopren® 6950 vertrieben werden.

Als weitere übliche Hilfs- und Zusatzstoffe können in der erfindungsgemäßen Zusammensetzung auch nichtionische und/oder kationische Tenside in Mengen von 0 bis 20 Gew.-%, vorzugsweise 0 bis 10 Gew.-%, eingesetzt werden. Derartige Tensidtypen sind beispielsweise quaternäre Ammoniumverbindungen, insbesondere aber quaternäre Ammoniumsalze, wie sie beispielsweise in den US-Patenten 5312522, 5397435, 5405501, 5427696 sowie den Patentanmeldungen EP-A 688901, WO95/11344, WO95/11343 WO95/01478, WO95/01479 WO94/29521, WO94/29520, WO94/16143 sowie WO94/19381 beschrieben sind.

Darüber hinaus können die erfindungsgemäßen Zusammensetzungen Riechstoffe üblicher Art enthalten, die ausgewählt sind aus natürlichen, naturidentischen oder künstlichen Riechstoffen (s. Def. in Römpp, 9. Aufl. 1992, S. 3887 f), wobei die entsprechenden Duftstoffe bevorzugt sind. Beispielsweise werden hier Agrumenöle wie Citronenöl, Bergamottöl, Orangenöl, Petit Grain-ÖI, Nadelholzöle, Foin Coupé-Riechstoffzusammensetzungen oder Blütenöle wie beispielsweise Rose, Jasmin, Flieder, Lavendel ebenso wie synthetische Duftstoffe auf Basis von Menthol usw. eingesetzt. Eine Übersicht hierüber gibt Ullmann, Enzyklopädie der technischen Chemie, Band 20, S. 190 - 285.

Darüber hinaus können auch zusammen mit der erfindungsgemäßen Zusammensetzung entsprechende anorganische Pigment- oder organische Farbstoffe zugesetzt werden, wie sie üblicherweise bei der Tissue- und non-woven-Papiererzeugung eingesetzt werden. Hierbei sind nicht zuletzt aus ökologischen Gründen physiologisch unbedenkliche und nicht hautirritierende Farbstoffe, insbesondere die entsprechenden natürlichen bzw. naturidentischen Farbstoffe, bevorzugt. Alle vorgenannten Zusatz- und Hilfsstoffe können sowohl einzeln als auch in Kombination enthalten sein.

Darüber hinaus können in der erfindungsgemäßen Zusammensetzung auch anorganische und/oder organische Füllstoffe enthalten sein, wie sie üblicherweise bei der Herstellung derartiger Produkte eingesetzt werden, wie beispielsweise Talkum, Bentonit und sonstige Tone.

Die Menge der so zugesetzten Füllstoffe beträgt üblicherweise 1 bis 50 Gew.-%, vorzugsweise aber 1 bis 10 Gew.-%.

Sofern erwünscht, können der erfindungsgemäßen Zusammensetzung auch Gelbildner oder Hydrogele zugesetzt werden, wie sie in Form natürlich vorkommender Polymere bekannt sind, beispielsweise auf Basis von Polysacchariden, Lignin, Naturkautschuk, Protein und/oder Naturharzen. Sofern hier Polysaccharide eingesetzt werden, so versteht man hierunter die aus Landpflanzen, aus Meerespflanzen oder aus Mikroorganismen gewonnenen, filmbildenden Materialien. Unter derartigen Gelbildnern auf Basis von Polysacchariden aus Landpflanzen versteht man üblicherweise Stärke und Stärkeprodukte, also beispielsweise Mais-, Weizen- und Reisstärke sowie Kartoffel- und Tapiokastärke. Weitere aus Landpflanzen gewonnene Polysaccharide sind die Galaktomannane, beispielsweise Johannisbrotkernmehl und Guar, Dipektine und Pektinstoffe, Exydatgummi wie Gummi arabicum oder Akaziengummi sowie Traganth, Cellulosederivate usw.

Bei den aus Meerespflanzen gewonnenen Polysacchrariden versteht man zunächst Alginate und Agar, Carragenane sowie Algenpolysaccharide. Aus Mikroorganismen erhaltene Polysaccharide sind schließlich das Dextran und das Xanthan. Gelbildner auf Basis von Proteinen, die in den erfindungsgemäßen Zusammensetzungen eingesetzt werden können, sind in der Hauptsache Gelatine, die üblicherweise als Typ A und Typ B im Handel angeboten werden. Unter synthetisch modifizierten, natürlich vorkommenden Polymeren, die in den erfindungsgemäßen Zusammensetzungen als verdickende Komponente eingesetzt werden können, versteht man die entsprechenden Celluloseether, Celluloseester, Stärkeester und Stärkeether und deren Mischungen. Schließlich können im Rahmen der erfindungsgemäßen Zusammensetzungen auch synthetische Polymere eingesetzt werden, die ausgewählt sind aus Polyvinylverbindungen, vorzugsweise Polyvinylalkohol, Polyvinylpyrrolidon oder Polyvinylbutyral, aus Polyacrylverbindungen, vorzugsweise Polymethacrylsäureestercopolymerisaten. Diese Komponenten können der Zusammensetzung in Mengen von 0 bis 40 Gew.-%, vorzugsweise 1 bis 20 Gew.-%, zugesetzt werden.

Eine Übersicht über derartig einzusetzende, wasserlösliche Polymere findet man in der Encyclopedia of Polymer Science and Engineering, Band 17 (1989), Seite 730 - 784.

Die vorgenannte erfindungsgemäße Zusammensetzung weist üblicherweise einen pH-Wert im Bereich von 3 bis 8 , vorzugsweise aber 3,5 bis 5 auf.

Die erfindungsgemäße Zusammensetzung weist üblicherweise bei den Applikationstemperaturen zwischen 10 und 90 °C, vorzugsweise 15 bis 80 °C, bei einem Sprühauftrag eine Viskosität von 1 bis 700 mPas, vorzugsweise 5 mPas bis 600 mPas und bei einem Walzenauftrag, Rakelauftrag oder Bladeauftrag eine Viskosität von 5 bis 2.000 mPas, vorzugsweise aber 10 mPas bis 1.500 mPas auf.

Bei einer bevorzugten Ausführungsform weist die erfindungsgemäße Zusammensetzung folgende Komponenten in folgenden Mengen auf: 30 bis 90 Gew.-%, vorzugsweise 50 bis 80 Gew.-% wenigstens eines Feuchtigkeitsregulators, insbesondere Glycerin und/oder Propylenglykol, 0 bis 20 Gew.-%, vorzugsweise 2 bis 10 Gew.-% wenigstens eines nichtionischen und/oder kationischen Tensids, 1 bis 25 Gew.-%, vorzugsweise 2 bis 20 Gew.-% wenigstens eines speziellen nichtionischen Tensids, ausgewählt aus Aminoxid und/oder Hydroxysäureestern sowie wenigstens eines Amphotensids, 0,01 bis 40 Gew.-%, vorzugsweise 0,01 bis 20 Gew.-% wenigstens eines Hautpflegemittels, 0 bis 70 Gew.-%, vorzugsweise 0 bis 40 Gew.-% wenigstens eines natürlichen, natürlich modifizierten oder synthetischen Gelbildners und Wasser als Rest.

Gegenstand der vorliegenden Erfindung ist weiterhin ein Verfahren zur Herstellung von Tissueprodukten der vorgenannten Art, welches dadurch gekennzeichnet ist, daß man eine Zusammensetzung der vorstehenden Art auf das Faservlies oder die Tissuebahn innerhalb der Siebpartie, Pressenpartie, TAD-Partie, am Yankee-Zylinder und/oder Trockenpartie, d. h. bei einer Faserstoffdichte von 20 bis 97 %, bezogen auf das Trockenfasergewicht der Bahn, in einer Menge von 0,1 bis 40 %, vorzugsweise 1 bis 20 %, kontinuierlich oder diskontinuierlich auf oder in der Bahn appliziert und die Bahn gegebenenfalls nach der Applikation einer Nachglättung unterzieht.

Nach einer alternativen Ausführungsform betrifft die vorliegende Erfindung ein Verfahren zur Herstellung von Tissueprodukte, dadurch gekennzeichnet, daß man eine Zusammensetzung der vorgenannten Art auf das Faservlies oder die Tissuebahn nach der Trockenpartie an der Wattemaschine, der Doubliermaschine und/oder beim Verarbeitungsautomaten in einer Menge von 0,1 bis 40 Gew.-%, vorzugsweise 1 bis 20 Gew.-%, kontinuierlich oder diskontinuierlich auf oder in der Bahn appliziert und die Bahn gegebenenfalls nach der Applikation einer Nachglättung unterzieht.

Nach einer bevorzugten Ausführungsform des erfindungsgemäßen Verfahrens wird die Zusammensetzung bei einer Faserstoffdichte von 35 bis 97 %, bezogen auf das Trockenfasergewicht der einlagigen Bahn in einer Menge von 0,2 bis 50%, vorzugsweise aber 1 bis 20 % appliziert.

Nach einer weiteren bevorzugten Ausführungsform des erfindungsgemäßen Verfahrens ist die Tissuebahn eine mehrlagige Bahn, und die Zusammensetzung wird bei einer Faserstoffdichte von mehr als 90 %, bezogen auf das Trockenfasergewicht, auf und/oder in wenigstens eine der Außenlagen der mehrlagigen Bahn in einer Menge von 0,001 bis 50 %, nachgewiesen als Einzelkomponente, z. B. über Headspace-Gaschromatographie, vorzugsweise 0,01 bis 30 %, oder weiter bevorzugt 0,1 bis 25%, appliziert, besonders bevorzugt 0,5 bis 20%.

Nach einer weiteren bevorzugten Ausführungsform des erfindungsgemäßen Verfahrens wird die Zusammensetzung auf und/oder in die mehrlagige Tissuebahn auf beiden Außenlagen in einer Menge von 0,001 bis 50 %, vorzugsweise 0,01 bis 30 %, appliziert.

Nach einer weiteren bevorzugten Ausführungsform des erfindungsgemäßen Verfahrens geschieht die Aufbringung der Zusammensetzung in der Tissueerzeugungsmaschine durch Sprühauftrag auf den Pope-Roller unter Erzeugung eines Behandlungsmittelfilms und dessen anschließlichen Transfers auf die Tissuebahn, der entweder während des Aufrollvorgangs oder der Auftrag durch Rakeln oder mittels des Blade-Verfahrens erfolgt. Auch ein Aufbringen durch Walzen, Sprühen, Rakeln oder das Blade-Verfahren ist möglich.

Alternativ kann die Aufbringung der Zusammensetzung, insbesondere aber des Hautpflegemittels in die Tissuebahn in mikroverkapselter Form, eingebettet in Mikrosponges oder in Form von Liposomen erfolgen.

Nach einer weiteren bevorzugten Ausführungsform des erfindungsgemäßen Verfahrens findet in den Fällen, in denen kein Auftrag in mikroverkapselter Form, eingebettet in Mikrosponges und in Form von Liposomen auf/in den Lagen stattfindet, eine Nachglättung durch wenigstens einen Durchgang der Tissuebahn durch einen Spalt eines Walzenpaares statt, bei dem eine Walze mit einer Stahloberfläche einer Gegenwalze mit einer Stahl-, Kunststoff-, Papier- oder Gummioberfläche, vorzugsweise einer Kunststoffoberfläche, zugeordnet ist. Im Rahmen dieser Verfahrensmodifikation ist es vorteilhaft, die Nachglättung durch einen zweimaligen Durchgang der Bahn durch einen Spalt eines Walzenpaares durchzuführen, bei dem zuerst eine Walze mit einer Stahloberfläche einer

Gegenwalze mit einer Kunststoffoberfläche und dann spiegelbildlich eine Walze mit einer Kunststoffoberfläche einer Walze mit einer Stahloberfläche zugeordnet ist.

Nach einer weiteren bevorzugten Ausführungsform des erfindungsgemäßen Verfahrens geschieht die Aufbringung der vorgenannten Zusammensetzung auf dem Faservlies im Rahmen eines konventionellen Faserherstellungs- oder Prozesses. Alternativ kann die Aufbringung der Zusammensetzung auf dem Faservlies auch im Rahmen einer Durchströmtrocknung bzw. eines TAD-Verfahrens erfolgen.

Gegenstand der vorliegenden Erfindung ist weiterhin die Verwendung der vorgenannten Zusammensetzung für die Behandlung von Tissueprodukten, insbesondere Taschentüchern, Kosmetiktüchern, Abschminktüchern, Toilettenpapier und Küchentüchern.

Weiterer bevorzugter Gegenstand der vorliegenden Erfindung ist ein Tissueprodukt enthaltend eine Feuchtigkeitsregulator enthaltende Zusammensetzung, welches dadurch gekennzeichnet ist, daß es 0,05 bis 50 Gew.-%, vorzugsweise 1 bis 30 Gew.-%, bezogen auf das Tissueprodukt, wenigstens ein spezielles nichtionisches Tensid, ausgewählt aus Aminoxid und/oder Hydroxysäureestem wobei Myristyllactat und Natriumsalze von Fettalkohollactaten ausgenommen sind und/oder wenigstens ein Amphotensid enthält.

Bei dem vorgenannten erfindungsgemäßen Tissueprodukt kann es sich um ein wenigstens einlagig, vorzugsweise zwei- bis vierlagig oder mehrlagiges, ungeprägtes, geprägtes oder teilgeprägtes, beispielsweise ein mit dekorativer Randprägung versehenes Tissuepapier, handeln. Anstelle von reinen Cellulosefasern können auch Tissuepapiere mit ungeprägten Mittellagen sowie einer Mittellage aus CTMP eingesetzt werden. Sofern ein mehrlagiges Tissuepapier eingesetzt wird, kann eine entsprechende Lagenhaftung erzeugt werden durch flächiges Verleimen, durch partielles Verleimen, beispielsweise punktuelles Verleimen nach einem vorgegebenen Muster, durch Zusammenpressen mittels einer Stahl-Stahl-Walze unter Druck durch eine mechanische Verhaftung der Einzellagen über die Flächen, mittels einer Rasterung, mittels einer Randprägung, mittels einer Rändelung usw. Bei einem derartigen Tissueprodukt kann es sich um ein voluminöses Trägermaterial mit einer ausreichend hohen Festigkeit und höchstmöglicher Weichheit handeln, die beispielsweise unter Einsatz von Tissue, non-woven oder einer Kombination beider Materialien erreicht werden kann. Hierbei setzt man beim Tissue Produkte mit einem Flächengewicht von etwa 15 bis 75 g/m² ein. Bei non-woven-Produkten geht man von einem Flächengewicht von 18 bis 120 g/m² aus. Hierbei können entweder Tissue- und non-woven-Produkte allein oder eine Kombination aus beiden mit jeweils unterschiedlichen Flächengewichten eingesetzt werden. Durch den Einsatz von non-wovens kann die Kompressibilität gewährleistet werden.

Soweit dies erwünscht ist, kann das entsprechende Trägermaterial auch naß verfestigt sein, wobei die üblichen, gesundheitlich unbedenklichen Naßfestmittel, beispielsweise Harnstoff- und Melaminharze sowie vernetzte kationische Polyalkylenamine usw. verwendet werden.

Nach einer bevorzugten Ausführungsform wird das erfindungsgemäße Tissueprodukt auf wenigstens einen Teil seiner Oberfläche, auf seinen Außenseiten, auf den Innenseiten der Außenlagen, nur auf den Innenlagen oder auf allen Lagen eine Zusammensetzung der vorgenannten Art aufweisen.

Nach einer weiteren bevorzugten Ausführungsform des erfindungsgemäßen Tissueprodukts findet man auf wenigstens einem Teil der vorgenannten Zusammensetzung als Auftrag in mikroverkapselter Form, eingebettet in Mikrosponges oder in Form von Liposomen, auf/in den Lagen, vorzugsweise aber wenigstens einen Teil des Hautpflegemittels der vorgenannten Art in mikroverkapselter Form, eingebettet in Mikrosponges oder in Form von Liposomen, auf bzw. in den Lagen.

Die vorgenannte Zusammensetzung weist als Grundbestandteil zunächst neben dem erfindungsgemäßen Tensid die Feuchtigkeitsregulatoren und Wasser als Rest auf. Als derartige Feuchtigkeitsregulatoren fungieren Polyole wie beispielsweise Polyethylenglykol oder Sorbit oder auch sonstige haut- und schleimhautverträgliche Substanzen. Hierbei ist es bevorzugt, Propylenglykol und/oder Glycerin einzusetzen.

Bezüglich der Hautpflegemittel wird besonders bevorzugt Provitamin B5, das sogenannte D-Panthenol, wegen seines sehr guten Feuchthaltevermögens eingesetzt. Weitere besonders bevorzugte Hautpflegekomponenten sind das Altantoin und die Additionsverbindung von Allantoin an D-Panthenol, das Tocopherolacetat, Jojobaöl, Reiskeimöl, Avocadoöl, spezielle Milchsäure-Fettalkoholester, beispielsweise das von der Merck AG herausgebrachte Produkt Ceraphyl® 28 H(CH₃)C(OH)C(O)OC₁₆H₃₃, Phytosterol, Glycyrrhithinsäure und deren Salze, die von der Firma Nordmann & Rassmann herausgebrachten Isopropylester von Wollwachsfettsäuren, beispielsweise das unter der Bezeichnung Amerlate® P herausgebrachte Isopropylmyristat, Bisabolol und Azulen, pflanzliche Proteine, wie beispielsweise Weizenproteine und Algenextrakte sowie verschiedene Pflanzenextrakte bzw. Wirkstoffe aus Hopfen, Johanniskraut, Melisse, Arnika, Eibisch, Holunder, Salbei, Malve, Hamamelis, Mango, Papaya und Linde.

Als Vertreter der erfindungsgemäßen Aminoxide, Hydroxysäureester und Amphotenside besonders bevorzugten Stoffklassen werden in den erfindungsgemäßen Zusammensetzungen eingesetzt Aminoxide der Formel 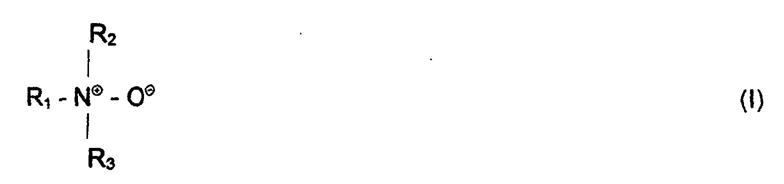 in der R₁ und R₂ Methyl ist und R₃ eine Amidoalkylgruppe der allgemeinen Formel (CH₂)₃N(H)C(O)R₄ ist, worin R₄ einen C7 bis C17-Fettsäurerest darstellt. Weitere besonders bevorzugte Amphotenside sind die sogenannten Alkylamphoacetate der allgemeinen Formel R₈(O)C(H)N(CH₂)₂(H)NCH₂(OH)CCH₂COO⁻, worin R₈ ein Fettsäurerest mit 7 bis 17 C-Atomen darstellt. Weiterhin sehr bevorzugt sind Alkylpolyamphopolycarboxyglycinate (APAC), bei denen wiederum 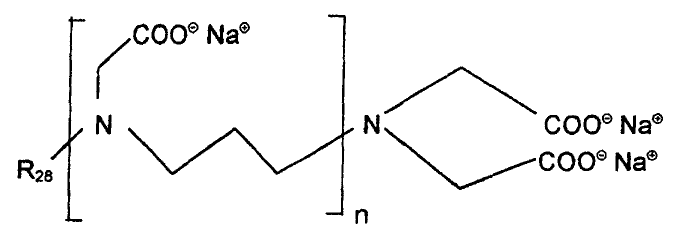 R₂₈ = C₇ - C₁₇-Fettsäure
n = 2,3 ist.

Weiter sind hier das Trimethylglycin zu nennen, welches die allgemeine Formel (CH₃)₃N⁺CH₂COO⁻ besitzt, ein Cocoamidopropylbetain oder ein Citronensäuremonoglycerid mit einem C7 bis C17-Fettsäurerest ist, bei der eine der beiden freien Citronensäurecarboxylgruppen in β-Stellung eine SO₃₋Gruppe aufweist.

Weiter zu nennen sind Glycinester der allgemeinen Struktur 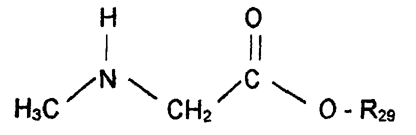 R₂₉ = C₇ - C₁₇ ⇒ -C₁₂H₂₅ (konkret: Medialan® LD, Bsp. 5)

Die vorgenannte Zusammensetzung dringt bei ihrer Anwendung zum Teil in das Tissuefasergewebe ein und beeinflußt so Weichheit und Haptik des Tuches. Bei Auftragungen ausschließlich auf die äußere Lage bleibt auf der oberen Schicht genügend Lotion haften, die bei ausreichendem Gebrauch der lotionierten Tücher auf die Haut übertragen wird, um dort ihre Wirkung entfalten zu können.

Zur Beurteilung der verbesserten/veränderten Haptik gegenüber Standards wurden sogenannte Handfeel-Tests durchgeführt. Dies geschieht in einem Panel-Test von geschulten Personen in Anlehnung an das Manual von Sensory Testing Methods, (ISTN, Special Technical Publication 434, S. 22, Testform de-ranking methods, rank order elevents printings, February 1993). Hiernach wird die steigende Weichheit, hier definiert als die Summe aus Oberflächensanftheit und Knüllweichheit, von einer Personengruppe von vier geschulten Personen mit nachfolgendem Verfahren bewertet:

Die zu prüfenden Papiertaschentücher waren zweimal hälftig gefaltet, so daß die Probenkennung für die Testperson nicht erkennbar war und in jedem Fall die gleiche Außenseite der Bewertung dargeboten wird.

Die solchermaßen geforderten Tücher werden dann zwischen Daumen, Daumenballen und Finger reibend und knüllend hinsichtlich ihrer Knüllweichheit und der Oberflächenweichheit geprüft und daraufhin mit vorher definierten Standards verglichen. Während die "Nullprobe", d. h. ein unbehandeltes Taschentuch aus der Standardproduktion einen Handfeel-Wert von nur 72 Handfeelpunkten auf einer Skala von 0 bis 100 aufweist, liegen die im Labor lotionierten Tücher stets darüber. Die nachfolgende Tabelle gibt einen Überblick über den Weichheitsgewinn.

Die vorliegende Erfindung wird nunmehr durch Ausführungsbeispiele näher erläutert.

### Beispiel 1

Einer Basislotion von 40 Gew.-% Glycerin, 30 Gew.-% Propylenglykol und 20 Gew.-% Wasser werden 10 Gew.-% des Citronensäurealkylpolyglykolestersulfosuccinat; Dinatriumsalz (Rewopol SB CS 50 der REWO GmbH) zugesetzt. Die Zugabe erfolgt unter Rühren, wobei eine gute Durchmischung gewährleistet sein muß. Es bildet sich eine klare Zusammensetzung in Form einer Lotion, die auf vierlagiges, randgeprägtes Tissuepapier mit einem Flächengewicht von 60 g/m² mit einem 6-%igen Auftrag appliziert wird. Nach 24-stündiger Konditionierung werden die Tücher nachgeglättet und in einem Panel-Test gegen andere Proben in Reihe gelegt. Das lotionierte Tissue wurde immer als weicher eingestuft als die unlotionierte Probe, sowohl eine nachgeglättete als auch eine ungeglättete.

### Beispiel 2

Einer Basislotion von 40 Gew.-% Glycerin, 30 Gew.-% Propylenglykol und 20 Gew.-% Wasser werden 10 Gew.-% eines Cocoamidoalkyldimethylaminoxids, wie es von der Firma Th. Goldschmidt AG unter der Bezeichnung Tegotain WS 35 vertrieben wird, zugesetzt. Die Zugabe erfolgt unter Rühren, wobei eine gute Durchmischung gewährleistet sein muß. Es bildet sich eine klare Lotion, die bei einem 6-%igen Auftrag auf vierlagiges, randgeprägtes Tissuepapier, wie in Beispiel 1 beschrieben, aufgetragen wird. Nach 24stündiger Konditionierung werden die Tücher nachgeglättet und in einem Panel-Test gegen andere Proben in Reihe gelegt. Das lotionierte Tissue schnitt in jedem Fall besser ab als ein nichtlotioniertes, nachgeglättetes oder auch ungeglättetes Tuch.

### Beispiel 3

Einer Basislotion von 50 Gew.-% Propylenglykol und 20 Gew.-% Wasser werden 10 Gew.-% des Aminoxids gemäß Beispiel 2 zugesetzt und unter Rühren gut gemischt. Anschließend werden 20 Gew.-% D-Panthenol (BASF AG) zugegeben. Die Zugabe erfolgt unter kräftigem Rühren, so daß eine gute Durchmischung sichergestellt ist. Nachdem sich eine einheitliche Lotion gebildet hat, wird sie in einem 6-%igen Auftrag auf ein vierlagiges, randgeprägtes Tissuepapier, wie in Beispiel 1 beschrieben, appliziert. Nach 24 Stunden Konditionieren glättet man das Tuch nach und prüft es ebenfalls innerhalb eines Panel-Tests. Das lotionierte Tissuepapier wurde wieder als besser eingestuft als ein unlotioniertes Tissue (ungeglättet oder nachgeglättet).

### Beispiel 4

Einer Basislotion von 30 Gew.-% Glycerin und 20 Gew.-% Propylenglykol und 29,75 Gew.-% Wasser werden unter Rühren 5,25 Gew.-% Aminoxid gemäß Beispiel 2 zugegeben. Anschließend fügt man noch 15 Gew.-% Phytosterol (Henkel KGaA) hinzu. Nach guter Durchmischung wird die Lotion in einem 6-%igen Auftrag auf ein vierlagiges, randgeprägtes Tissuepapier gemäß Beispiel 1 appliziert.

Nach 24 Stunden Konditionieren und Nachglätten wurde das lotionierte Tuch in einem Panel-Test immer besser eingestuft als ein nichtlotioniertes Tuch.

### Beispiel 5

Einer Basislotion von 46 Gew.-% Propylenglykol und 20 Gew.-% Wasser werden unter Rühren als Tensid 2 Gew.-% des Aminoxids gemäß Beispiel 2 sowie 2% Medialan® LD und als Hautpflegemittel 3 Gew.-% Tocopherolacetat, 12 Gew.-% Reiskeimöl, und 15 Gew.-% Jojobaöl zugegeben. Nach guter Durchmischung wird die Lotion in einem 6-%igen Auftrag auf ein vierlagiges, randgeprägtes Tissuepapier gemäß Beispiel 1 appliziert. Nach 24 Stunden Konditionieren und Nachglätten wurde das lotionierte Tuch in einem Panel-Test immer besser eingestuft als ein nichtlotioniertes Tuch.

### Beispiel 6

Beispiel 2 wurde wiederholt, wobei allerdings anstelle des Aminoxids 10 Gew.-% des Alkylamphoacetates Rewoteric AM 2C NM (Fa. Witco Surfactants), 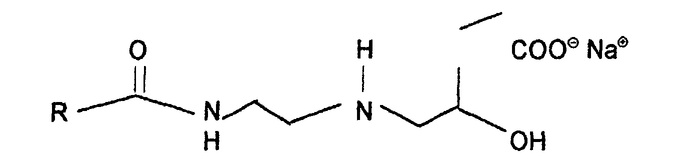 R = C₇ - C₁₇-Fettsäure
zugegeben worden sind. Bei sonst gleicher Handlungsweise fand man im Anschluß an die Auftragung und die Konditionierung sowie die Nachglättung in einem Panel-Test, daß das lotionierte Tissue in jedem Fall besser abschnitt als das entsprechend nichtlotionierte.

### Beispiel 7

Einer Basislotion aus 69 Gew.-% Propylenglykol und 20 Gew.-% Wasser werden 10 Gew.-% des Aminoxids gemäß Beispiel 2 zugesetzt und unter Rühren gut gemischt. Anschließend fügt man unter Erwärmung auf ca. 60 °C und kräftigem Rühren 1 Gew.-% Glycyrrhitinsäure (Midas Pharma GmbH, Ingelheim) hinzu. Nach dem Auflösen und anschließendem Abkühlen auf 20 °C wird die Lotion in einem 6-%igen Auftrag auf ein vierlagiges, randgeprägtes Tissuepapier gemäß Beispiel 1 appliziert. Nach 24 Stunden Konditionieren und Nachglätten wurde das lotionierte Tuch in einem Panel-Test besser eingestuft als ein nicht lotioniertes Tuch.

### Beispiel 8

Beispiel 2 wurde wiederholt mit der Maßgabe, daß anstelle des Aminoxids 10% Trimethylglycin (Fa. WITCO) zugegeben worden sind. Bei ansonsten gleicher Verfahrensdurchführung erhielt man eine klare Lotion, die in einem 6-%igen Auftrag auf ein vierlagiges, randgeprägtes Tissuepapier gemäß Beispiel 1 appliziert worden ist. Nach 24-stündigem Konditionieren und Nachglätten wurde das lotionierte Tuch in einem Panel-Test besser eingestuft als ein nicht lotioniertes Tuch.

### Beispiel 9

Zu einer Basislotion, bestehend aus Propylenglykol, Glycerin und Wasser (Mengenverhältnis 30 : 45 : 25) wurden 4% eines Bentonits (Optigel® SH, Süd-Chemie AG) zugesetzt. Die Viskosität der so behandelten Basislotion wurde auf etwa 840 mPas angehoben, um einen Auftrag mittels Filmpressen zu ermöglichen. Diese

Lotion wurde 6-%ig beidseitig mittels Sprühverfahren appliziert und ergab einen Handfeel-Wert von 77 bei einer Schwankungsbreite von +- 5.

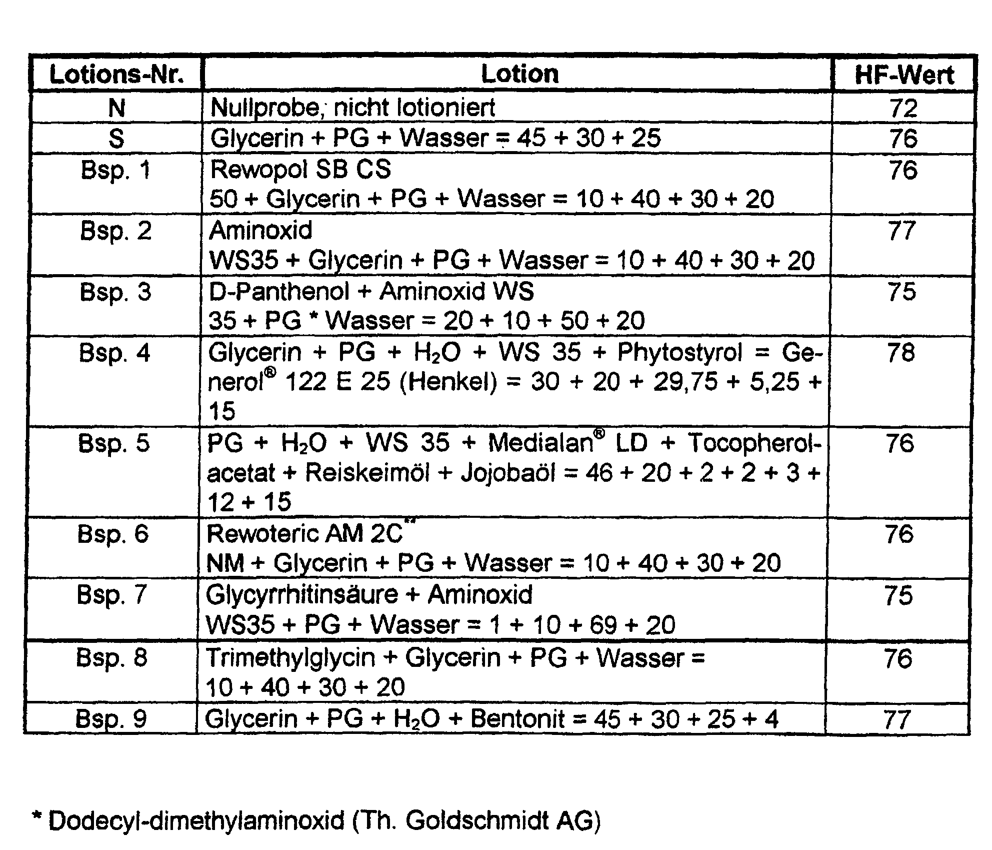

## Patentansprüche

1. Feuchtigkeitsregulator enthaltende Zusammensetzung für Tissueprodukte, **dadurch gekennzeichnet, daß** sie weiterhin 1 bis 25 Gew.-%, vorzugsweise 2 bis 20 Gew.-%, bezogen auf die Gesamtzusammensetzung, wenigstens eines speziellen nichtionischen Tensids ausgewählt aus wenigstens einem Aminoxid und/oder Hydroxysäureestern, ausgenommen Myristyllactat und Natriumsalze von Fettalkohollactaten, und / oder wenigstens eines Amphotensids enthält und wobei der Feuchtigkeitsregulator in einer Menge von 40 bis 90 Gew.-%, bezogen auf die Gesamtzusammensetzung, enthalten ist.

2. Zusammensetzung nach Anspruch 1, **dadurch gekennzeichnet, daß** das Aminoxid die allgemeine Formel 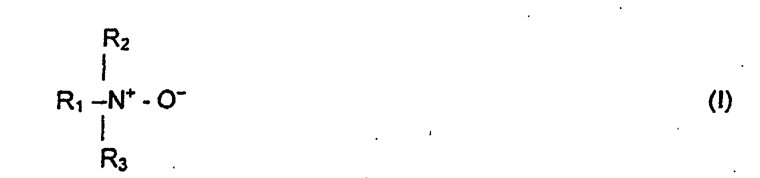 aufweist, in der R₁, R₂ und R₃, unabhängig voneinander, für einen gegebenenfalls substituierten aliphatischen linearen oder verzweigten Alkylrest mit 1 bis 25 C-Atomen, für einen gegebenenfalls substituierten cyclischen Alkylrest mit 3 bis 22 C-Atomen, für einen gegebenenfalls substituierten aliphatischen linearen oder verzweigten Amidoalkylrest mit 3 bis 22 C-Atomen im Alkylteil oder für einen gegebenenfalls substituierten cyclischen Amidoalkylrest mit 3 bis 10 C-Atomen im Alkylteil steht.

3. Zusammensetzung nach Anspruch 2, **dadurch gekennzeichnet, daß** das Aminoxid eine allgemeine Formel (I) aufweist, in der R₁ und R₂ eine gegebenenfalls substituierte Alkylgruppe mit 1 bis 4 C-Atomen, vorzugsweise Methyl, ist und R₃ ein Amidoalkylrest der allgemeinen Formel
(CH₂)ₐN(H)C(O)R₄ (II)
ist, bei dem a 1 bis 5 ist und R₄ ein aus natürlichen Ölen und Fetten stammende Fettsäurerest mit 7 bis 25 C-Atomen, vorzugsweise 7 bis 17 C-Atomen ist.

4. Zusammensetzung nach Anspruch 1, **dadurch gekennzeichnet, daß** der Hydroxysäureester ein Fruchtsäure(halb)ester, Milchsäureester, Ricinolsäureester oder ein Glucosesäureester, vorzugsweise ein Citronensäuremono-, di- oder -triester ist, der wenigstens einen aus natürlichen Ölen und Fetten stammenden Fettsäurerest mit 1 bis 25 C-Atomen, vorzugsweise 7 bis 17 C-Atomen enthält, der gegebenenfalls weiter eine polare Gruppen enthält.

5. Zusammensetzung nach Anspruch 1, **dadurch gekennzeichnet, daß** das Amphotensid ein Ampholyt oder ein Betain ist.

6. Zusammensetzung nach Anspruch 5, **dadurch gekennzeichnet, daß** das Betain abgeleitet ist aus natürlichen oder synthetischen Quellen und ausgewählt ist aus Alkylbetainen der allgemeinen Formel
R₅(CH₃)N⁺(CH₃)CH₂COO⁻ (III),
wobei R₅ eine gegebenenfalls substituierte lineare oder verzweigte Alkylgruppe mit 1 bis 25 C-Atomen aufweist, Alkylamidobetainen der allgemeinen Formel
R₆(CO)NH(CH₂)₀(CH₃)N⁺(CH₃)CH₂COO⁻ (IV),
wobei R₆ eine gegebenenfalls substituierte lineare oder verzweigte Alkylgruppe mit 1 bis 25 C-Atomen ist, und ₒ gleich 1 bis 5 ist,
Sulfobetainen der allgemeinen Formel
R₇(CH₃)N⁺(CH₃)-X-SO₃⁻ (V),
wobei R₇ eine gegebenenfalls substituierte lineare oder verzweigte Alkylgruppe mit 1 bis 25 C-Atomen und X eine gegebenenfalls substituierte Alkylengruppe mit 1 bis 5 Methylen-Einheiten ist, Alkylpolyamphopolycarboxyglycinate der allgemeinen Formel 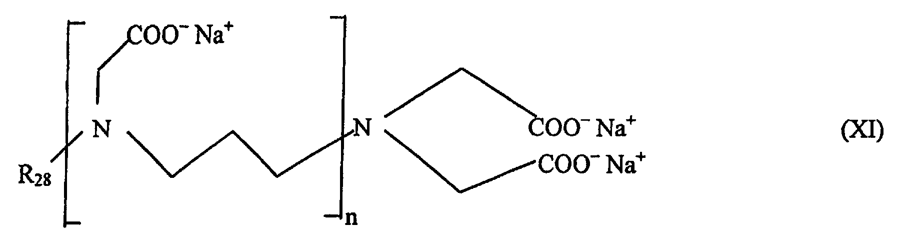 wobei R₂₈ eine C₇ - C₁₇-Fettsäure und n gleich 2 oder 3 ist oder Glycinester der allgemeinen Formel 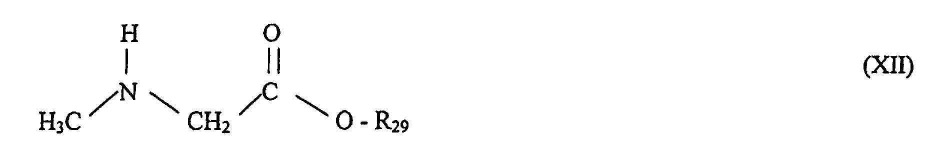 wobei R₂₉ eine C₇ - C₁₇-Alkylgruppe, vorzugsweise C₁₂H₂₅ ist.

7. Zusammensetzung nach vorstehenden Ansprüchen, **dadurch gekennzeichnet, daß** der Feuchtigkeitsregulator in einer Menge von 50 bis 80 Gew.-%, bezogen auf die Gesamtzusammensetzung, enthalten ist und insbesondere ausgewählt ist aus Polyolen.

8. Zusammensetzung nach vorstehenden Ansprüchen, **dadurch gekennzeichnet, daß** das Polyol ausgewählt ist aus Glycerin, Propylenglykol, Butylenglykol, Polyalkylenglykol und Zuckeralkoholen.

9. Zusammensetzung nach vorstehenden Ansprüchen, **dadurch gekennzeichnet, daß** sie weiterhin 0,01 bis 50 Gew.-% , vorzugsweise 0,1 bis 40 Gew.-% wenigstens eines natürlichen, naturidentischen oder synthetischen Hautpflegemittels, vorzugsweise auf natürlich pflanzlicher Basis, enthält.

10. Zusammensetzung nach vorstehenden Ansprüchen, **dadurch gekennzeichnet, daß** sie weiterhin übliche Hilfs- und Zusatzstoffe, wie nichtionische und/oder kationische Tenside, anorganische und/oder organische Füllstoffe, Farbstoffe und/oder natürliche oder synthetische Gelbildner enthält.

11. Zusammensetzung nach vorstehenden Ansprüchen **dadurch gekennzeichnet, daß** sie einen pH-Wert von 3 bis 8, vorzugsweise 3,5 bis 5 aufweist.

12. Zusammensetzung nach vorstehenden Ansprüchen, **dadurch gekennzeichnet, daß** sie bei einer Applikationstemperatur von 10 bis 90 °C bei einem Sprühauftrag eine Viskosität von 1 mPas bis 700 mPas, vorzugsweise 5 mPas bis 600 mPas und bei einem Walzauftrag, (Roll)Rakelauftrag oder Bladeauftrag eine Viskosität von 5 mPas bis 2.000 mPas, vorzugsweise 10 mPas bis 2.000 mPas, aufweisen.

13. Zusammensetzung nach vorstehenden Ansprüchen, enthaltend 40 bis 90 Gew.-%, vorzugsweise 50 bis 80 Gew.-% wenigstens eines Feuchtigkeitsregulators, 0 bis 20 Gew.-%, vorzugsweise 2 bis 10 Gew.-% wenigstens eines nichtionischen und/oder kationischen Tensids, 1 bis 25 Gew.-%. vorzugsweise 2 bis 20 Gew.-% wenigstens eines speziellen nichtionischen Tensids ausgewählt aus Aminoxid und/oder Hydroxysäureestern sowie wenigstens eines Amphotensids, 0,01 bis 40 Gew.-%, vorzugsweise 0,01 bis 20 Gew.-%, wenigstens eines Hautpflegemittels, 0 bis 70 Gew.-%, vorzugsweise 0 bis 40 Gew.-% wenigstens eines Gelbildners und Wasser als Rest.

14. Verfahren zur Herstellung von Tissueprodukten, **dadurch gekennzeichnet, daß** man eine Zusammensetzung nach Ansprüchen 1 bis 13 auf das Faservlies oder die Tissuebahn innerhalb der Siebpartie, Pressenpartie, TAD-Partie, am Yankee-Zylinder und oder Trockenpartie, d.h. bei einer Faserstoffdichte von 20 bis 97 %, bezogen auf das Trockenfasergewicht der Bahn, in einer Menge von 0,1 bis 50 Gew.-%, vorzugsweise 0,2 bis 50 Gew.-%, kontinuierlich oder diskontinuierlich auf oder in der Bahn appliziert und die Bahn gegebenenfalls nach der Applikation einer Nachglättung unterzieht.

15. Verfahren zur Herstellung von Tissueprodukten, **dadurch gekennzeichnet, daß** man eine Zusammensetzung nach Ansprüchen 1 bis 13 auf das Faservlies oder die Tissuebahn, nach der Trockenpartie an der Wattemaschine, der Doubliermaschine und/oder beim Verarbeitungsautomaten, in einer Menge von 0,1 bis 40 Gew.-%, vorzugsweise 1 bis 20 Gew.-%, kontinuierlich oder diskontinuierlich auf oder in der Bahn appliziert und die Bahn gegebenenfalls nach der Applikation einer Nachglättung unterzieht.

16. Verfahren nach Anspruch 15, **dadurch gekennzeichnet, daß** man die Zusammensetzung bei einer Faserstoffdichte von 35 bis 97 %, bezogen auf das Trockenfasergewicht der einlagigen Bahn, in einer Menge von 0,2 - 50 %, vorzugsweise 1 bis 20 %, appliziert.

17. Verfahren nach Ansprüchen 14 bis 16, **dadurch gekennzeichnet, daß** die Tissuebahn eine mehrlagige Bahn ist und die Zusammensetzung bei einer Faserstoffdichte von mehr als 90 %, bezogen auf das Trockenfasergewicht, auf wenigstens eine der Außenlagen der mehrlagigen Bahn in einer Menge von 0,001 bis 50 %, vorzugsweise 0,01 bis 30 %, appliziert wird.

18. Verfahren nach Ansprüchen 17, **dadurch gekennzeichnet, daß** die Zusammensetzung auf die mehrlagige Bahn in einer Menge von 0,001 bis 50 %, vorzugsweise 0,01 bis 30 %, appliziert wird.

19. Verfahren nach Ansprüchen 14 bis 18, **dadurch gekennzeichnet, daß** die Aufbringung wenigstens eines Teils der Zusammensetzung auf der Bahn durch Sprühen, Walzen, (Roll)Rakeln oder über das Blade-Verfahren oder in und / oder auf der Bahn Mikroverkapselung, eingebettet in Mikrosponges oder in Form von Liposomen, insbesondere bezüglich des Hautpflegemittels, erfolgt.

20. Verfahren nach Ansprüchen 14 bis 19, **dadurch gekennzeichnet, daß** die Nachglättung, außer nach dem Verfahren nach Anspruch 19, letzte Alternative, durch wenigstens einen Durchgang der Tissue-Bahn durch einen Spalt eines Walzenpaares erfolgt, bei dem eine Walze mit einer Stahloberfläche einer Gegenwalze mit einer Stahl-, Kunststoff-, Papier- oder Gummioberfläche, vorzugsweise einer Kunststoffoberfläche zugeordnet ist.

21. Verfahren nach Ansprüchen 14 bis 19, **dadurch gekennzeichnet, daß** die Nachglättung, außer nach dem Verfahren nach Anspruch 19, letzte Alternative, durch einen zweimaligen Durchgang der Tissue-Bahn durch einen Spalt eines Walzenpaares erfolgt, bei dem zuerst eine Walze mit einer Stahloberfläche einer Gegenwalze mit einer Kunststoffoberfläche und dann spiegelbildlich eine Walze mit einer Kunststoffoberfläche einer Gegenwalze mit einer Stahloberfläche zugeordnet ist.

22. Verfahren nach Ansprüchen 14 bis 21, **dadurch gekennzeichnet, daß** die Aufbringung der Zusammensetzung auf das Faservlies im Rahmen eines konventionellen Tissueherstellungsverfahrens erfolgt.

23. Verfahren nach Ansprüchen 14 bis 21, **dadurch gekennzeichnet, daß** die Aufbringung der Zusammensetzung auf das Faservlies im Rahmen einer Durchströmtrocknung (TAD-Verfahren) oder im Rahmen eines Non-woven-Vlieslegungsprozesses erfolgt.

24. Verwendung der Zusammensetzung nach Ansprüchen 1 bis 13, insbesondere in Lotionform für die Behandlung von Tissueprodukten, insbesondere Taschentüchern, Kosmetiktüchern, Abschminktüchern, Toilettentüchern und Küchentüchern, insbesondere nach dem Verfahren nach Ansprüchen 14 bis 23.

25. Tissueprodukt enthaltend eine Feuchtigkeitsregulator enthaltende Zusammensetzung, **dadurch gekennzeichnet, daß** die Feuchtigkeitsregulator enthaltende Zusammensetzung 1 bis 25 Gew.-%, vorzugsweise 2 bis 20 Gew.-% bezogen auf die Gesamtzusammensetzung, wenigstens eines speziellen nichtionischen Tensids ausgewählt aus wenigstens einem Aminoxid und/oder Hydroxysäureestern, ausgenommen Myristylacetat und Natriumsalze von Fettalkohollactaten, und/oder wenigstens eines Amphotensids enthält, und wobei der Feuchtigkeitsregulator in einer Menge von 40 bis 90 Gew.-% bezogen auf die Gesamtzusammensetzung enthalten ist.

26. Tissueprodukt nach Anspruch 25, **dadurch gekennzeichnet, daß** es 0,05 bis 50 Gew.-%, vorzugsweise 1 bis 30 Gew.-% bezogen auf das Tissueprodukt, eines speziellen nichtionischen Tensids ausgewählt aus wenigstens einem Aminoxid und/oder Hydroxysäureestern, ausgenommen Myristyllactat und Natriumsalze von Fettalkolhollactaten, und/oder wenigstens eines Amphotendsids.

27. Tissueprodukt nach Anspruch 25 und 26, **dadurch gekennzeich** **net, daß** es weiterhin auf wenigstens einem Teil seiner Oberfläche, auf seinen Außenseiten, auf den Innenseiten der Außenlagen, nur auf den Innenlagen oder auf allen Lagen, eine Zusammensetzung nach Ansprüchen 1 bis 13 aufweist.

28. Tissueprodukt nach Anspruch 25 bis 27 **dadurch gekennzeichnet, daß** wenigstens ein Teil der Zusammensetzung nach Ansprüchen 1 bis 13 als Auftrag, in mikroverkapselter Form, eingebettet in Mikrosponges oder in Form von Liposomen auf / in den Lagen vorliegt, vorzugsweise wenigstens ein Teil des Hautpflegemittel nach Anspruch 9 in mikroverkapselter Form, eingebettet in Mikrosponges oder in Form von Liposomen auf/in den Lagen vorliegt.

## Claims

1. A composition for tissue products containing moisture regulator, **characterised in that** it further contains 1 to 25 % by weight, preferably 2 to 20 % by weight, in relation to the total composition, of at least one special non-ionic surfactant chosen from at least one amine oxide and/or hydroxy acid esters, with the exception of myristyl lactate and sodium salts of fatty alcohol lactates, and/or at least one amphoteric surfactant, and wherein the moisture regulator is contained in a quantity of 40 to 90 % by weight, in relation to the total composition.

2. A composition according to Claim 1, **characterised in that** the amine oxide has the general formula 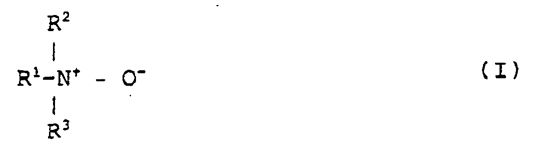 in which R¹,R² and R³, independently of one another, represent an optionally substituted aliphatic linear or branched alkyl residue with 1 to 25 C atoms, an optionally substituted cyclic alkyl residue with 3 to 22 C atoms, an optionally substituted aliphatic linear or branched amido alkyl residue with 3 to 22 C atoms in the alkyl portion or an optionally substituted cyclic alkyl residue with 3 to 10 C atoms in the alkyl portion.

3. A composition according to Claim 2, **characterised in that** the amine oxide has a general formula (I), in which R₁ and R₂ are an optionally substituted alkyl group with 1 to 4 C atoms, preferably methyl, and R₃ is an amido alkyl residue of the general formula
(CH₂)ₐN(H)C(O)R₄ (II),
wherein a is 1 to 5 and R₄ is a fatty acid residue with 7 to 25 C atoms, preferably 7 to 17 C atoms, originating from natural oils and fats.

4. A composition according to Claim 1, **characterised in that** the hydroxy acid ester is a fruit acid (semi) ester, lactic acid ester, ricinoleic acid ester or a glucose acid ester, preferably a citric acid mono, di or triester, which contains at least one fatty acid residue with 1 to 25 C atoms, preferably 7 to 17 C atoms, originating from natural oils and fats, which optionally also contains a polar group.

5. A composition according to Claim 1, **characterised in that** the amphoteric surfactant is an ampholyte or a betaine.

6. A composition according to Claim 5, **characterised in that** the betaine is derived from natural or synthetic sources and is chosen from alkyl betaines of the general formula
R₅(CH₃)N⁺(CH₃)CH₂COO⁻ (III),
wherein R₅ is an optionally substituted linear or branched alkyl group with 1 to 25 C atoms, alkyl amido betaines of the general formula
R₆(CO)NH(CH₂)₀(CH₃)N⁺(CH₃)CH₂COO⁻ (IV),
wherein R₆ is an optionally substituted linear or branched alkyl group with 1 to 25 C atoms, and ₀ is equal to 1 to 5, sulphobetaines of the general formula
R₇(CH₃)N⁺(CH₃)-X-SO₃⁻ (V),
wherein R₇ is an optionally substituted linear or branched alkyl group with 1 to 25 C atoms, and X an optionally substituted alkylene group with 1 to 5 methylene units, alkyl polyamphopolycarboxy glycinates of the general formula 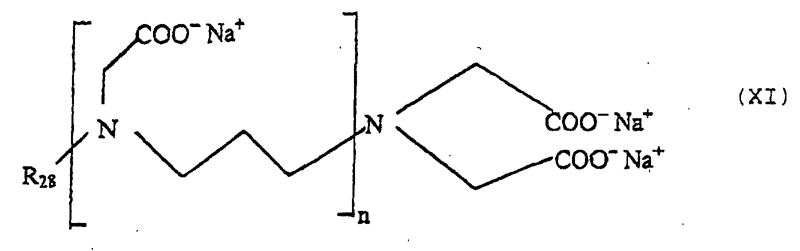 wherein R₂₆ is a C₇ - C₁₇ fatty acid and n is equal to 2 or 3 or glycine esters of the general formula 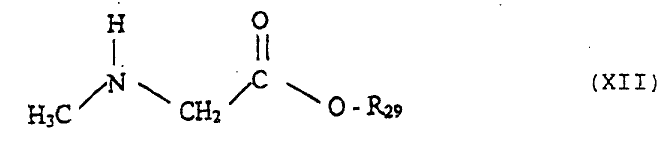 wherein R₂₉ is a C₇ - C₁₇ alkyl group, preferably C₁₂H₂₅.

7. A composition according to the preceding Claims, **characterised in that** the moisture regulator is contained in a quantity of 50 to 80 % by weight, in relation to the total composition, and in particular is chosen from polyols.

8. A composition according to the preceding Claims, **characterised in that** the polyol is chosen from glycerine, propylene glycol, butylene glycol, polyalkylene glycol and sugar alcohols.

9. A composition according to the preceding Claims, **characterised in that** it further contains 0.01 to 50 % by weight, preferably 0.1 to 40 % by weight, of at least one natural, nature-identical or synthetic skin care product, preferably with a natural vegetable base.

10. A composition according to the preceding Claims, **characterised in that** it further contains auxiliary agents and additives, such as non-ionic and/or cationic surfactants, inorganic and/or organic fillers, dyes and/or natural or synthetic gelling agents.

11. A composition according to the preceding Claims, **characterised in that** it has a pH value of 3 to 8, preferably 3.5 to 5.

12. A composition according to the preceding Claims, **characterised in that**, at an application temperature of 10 to 90°C with spray coating, it has a viscosity of 1 mPas to 700 mPas, preferably 5 mPas to 600 mPas, and with roller coating, (roll) doctor coating or blade application it has a viscosity of 5 mPas to 2,000 mPas, preferably 10 mPas to 2,000 mPas.

13. A composition according to the preceding Claims, containing 40 to 90 % by weight, preferably 50 to 80 % by weight of at least one moisture regulator, 0 to 20 % by weight, preferably 2 to 10 % by weight of at least one non-ionic and/or cationic surfactant, 1 to 25 % by weight, preferably 2 to 10 % by weight of at least one special non-ionic surfactant chosen from amine oxide and/or hydroxy acid esters, as well as at least one amphoteric surfactant, 0.01 to 40 % by weight, preferably 0.01 to 20 % by weight of at least one skin care product, 0 to 70 % by weight, preferably 0 to 40 % by weight of at least one gelling agent and the remainder water.

14. A method for the production of tissue products, **characterised in that** a composition according to Claims 1 to 13 is applied to the non-woven fabric or the tissue web within the wire section, press section, TAD section, on the Yankee cylinder and/or drying section, i.e. with a fibre density of 20 to 97 %, in relation to the dry fibre weight of the web, in a quantity of 0.1 to 50 % by weight, preferably 0.2 to 50 % by weight, continuously or discontinuously on or in the web and optionally after application the web is subjected to post-polishing.

15. A method for the production of tissue products, **characterised in that** a composition according to Claims 1 to 13 is applied to the non-woven fabric or the tissue web, after the drying section in the wadding machine, doubling machine and/or in automatic processing machines, in a quantity of 0.1 to 40 % by weight, preferably 1 to 20 % by weight, continuously or discontinuously on or in the web and optionally after application the web is subjected to post-polishing.

16. A method according to Claim 15, **characterised in that** the composition is applied with a fibre density of 35 to 97 %, in relation to the dry fibre weight of the single-layer web, in a quantity of 0.2 - 50 %, preferably 1 to 20 %.

17. A method according to Claims 14 to 16, **characterised in that** the tissue web is a multilayer web and the composition is applied with a fibre density of more than 90 %, in relation to the dry fibre weight, to at least one of the outer layers of the multilayer web in a quantity of 0.001 to 50 %, preferably 0.01 to 30 %.

18. A method according to Claim 17, **characterised in that** the composition is applied to the multilayer web in a quantity of 0.001 to 50 %, preferably 0.01 to 30 %.

19. A method according to Claims 14 to 18, **characterised in that** the application of at least one portion of the composition to the web is effected by spraying, rolling, (roll) doctoring or via the blade process or in and/or on the web by microencapsulation, embedded in microsponge or in the form of liposomes, particularly with regard to the skin care product.

20. A method according to Claims 14 to 19, **characterised in that**, apart from according to the method of Claim 19, last alternative, the post-polishing is carried out by at least one passage of. the tissue web through a gap in a pair of rollers, in which one roller having a steel surface is associated with a counter roller having a steel surface, plastics surface, paper or rubber surface, preferably a plastics surface.

21. A method according to Claims 14 to 19, **characterised in that**, apart from according to the method of Claim 19, last alternative, the post-polishing is carried out by passing the tissue web twice through a gap in a pair of rollers, in which firstly a roller having a steel surface is associated with a counter roller having a plastics surface and then in mirror image a roller having a plastics surface is associated with a counter roller having a steel surface.

22. A method according to Claims 14 to 21, **characterised in that** the application of the composition to the non-woven. fabric is carried within the framework of a conventional tissue production process.

23. A method according to Claims 14 to 21, **characterised in that** the application of the composition to the non-woven fabric is carried within the framework of a throughflow drying process (TAD process) or within the framework of a non-woven fabric laying process.

24. Use of the composition according to Claims 1 to 13, in particular in lotion form for the treatment of tissue products, in particular handkerchiefs, cosmetic tissues, makeup-removal tissues, toilet tissues and kitchen tissues, in particular according to the method of Claims 14 to 23.

25. A tissue product containing a composition containing moisture regulator, **characterised in that** the composition containing moisture regulator contains 1 to 25 % by weight, preferably 2 to 20 % by weight, in relation to the total composition, of at least one special non-ionic surfactant chosen from at least one amine oxide and/or hydroxy acid esters, with the exception of myristyl lactate and sodium salts of fatty alcohol lactates, and/or at least one amphoteric surfactant, and wherein the moisture regulator is contained in a quantity of 40 to 90 % by weight, in relation to the total composition.

26. A tissue product according to Claim 25, **characterised**
**in that** 0.05 to 50 % by weight, preferably 1 to 30 % by weight, in relation to the tissue product, of a special non-ionic surfactant chosen from at least one amine oxide and/or hydroxy acid esters, with the exception of myristyl lactate and sodium salts of fatty alcohol lactates, and/or at least one amphoteric surfactant.

27. A tissue product according to Claims 25 and 26, **characterised in that** on at least one portion of its surface, on its outer sides, on the insides of the outer layers, only on the inner layers or on all layers, it further has a composition according to Claims 1 to 13.

28. A tissue product according to Claims 25 to 27, **characterised in that** at least one portion of the composition according to Claims 1 to 13 is present as a coating, in microencapsulated form, embedded in microsponge or in the form of liposomes on/in the layers, preferably at least one portion of the skin care product according to Claim 9 is present in microencapsulated form, embedded in microsponge or in the form of liposomes on/in the layers.

## Revendications

1. Composition contenant un régulateur d'humidité pour articles en tissu ouate, **caractérisée en ce qu'**elle contient 1 à 25 % en poids, de préférence 2 à 20 % en poids, rapporté à la composition totale, d'au moins un agent de surface spécial non ionique, choisi entre au moins un oxyde d'amine et/ou des esters d'hydroxyacides, à l'exception du lactate de myristyle et des sels de sodium des lactates d'alcools gras, et/ou au moins un agent de surface ampholyte, le régulateur d'humidité étant contenu dans une quantité de 40 à 90 % en poids, rapportée à la composition totale.

2. Composition selon la revendication 1, **caractérisée en ce que** l'oxyde d'amine présente la formule générale 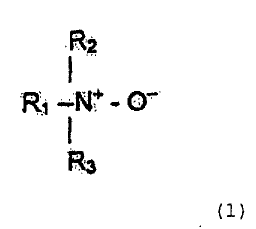 dans laquelle R₁, R₂ et R₃, indépendamment l'un de l'autre, représentent un reste alkyle linéaire ou ramifié, substitué le cas échéant, avec 1 à 25 atomes de C, un reste alkyle cyclique, substitué le cas échéant, avec 3 à 22 atomes de C, un reste amidoalkyle, substitué le cas échéant, avec 3 à 22 atomes de C dans la partie alkyle, ou un reste amidoalkyle cyclique, substitué le cas échéant, avec 3 à 10 atomes de C dans la partie alkyle.

3. Composition selon la revendication 2, **caractérisée en ce que** l'oxyde d'amine présente une formule générale (I), dans laquelle R₁ et R₂ sont des groupes alkyles, substitués le cas échéant, avec 1 à 4 atomes de C, de préférence des groupes méthyles, et R₃ est un reste amidoalkyle de la formule générale
(CH₂)ₐN(H)C(O)R₄ (II)
pour lequel a est un nombre compris entre 1 et 5 et R₄ est un reste d'acide gras provenant d'huiles et de graisses naturelles avec 7 à 25 atomes de C, de préférence 7 à 17 atomes de C.

4. Composition selon la revendication 1, **caractérisée en ce que** l'ester d'hydroxyacide est un (hémi)ester d'un acide de fruit, un ester de l'acide lactique, un ester de l'acide ricinoléique ou un ester de l'acide gluconique, de préférence un mono-, di- ou triester de l'acide citrique, qui contient au moins un reste d'acide gras de 1 à 25 atomes de C, de préférence 7 à 17 atomes de C, provenant d'huiles et de graisses naturelles, lequel contient le cas échéant d'autres groupes polaires.

5. Composition selon la revendication 1, **caractérisée en ce que** l'agent de surface ampholyte est un ampholyte ou une bétaïne.

6. Composition selon la revendication 5, **caractérisée en ce que** la bétaïne est dérivée de sources naturelles ou synthétiques, et est choisie parmi les alkyle bétaïnes de la formule générale
R₅(CH₃)N⁺(CH₃)CH₂COO⁻ (III)
dans laquelle R₅ est un groupe alkyle linéaire ou ramifié, substitué le cas échéant, de 1 à 25 atomes de C, parmi les alkylamidobétaïnes de la formule générale
R₆(CO)NH(CH₂)₀(CH₃)N⁺(CH₃)CH₂COO⁻ (IV)
dans laquelle R₆ est un groupe alkyle linéaire ou ramifié, substitué le cas échéant, de 1 à 25 atomes de C, et o un nombre compris entre 1 et 5,
parmi les sulfobétaïnes de la formule générale
R₇(CH₃)N⁺(CH₃)-X-SO₃⁻ (V)
dans laquelle R₇ est un groupe alkyle linéaire ou ramifié, substitué le cas échéant, de 1 à 25 atomes de C, et X un groupe alkylène, substitué le cas échéant, constitué de 1 à 5 unités méthylène, parmi des alkylpolyamphopolycarboxyglycinates de la formule générale 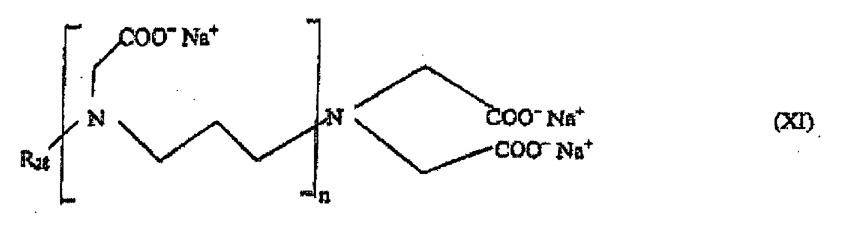 dans laquelle R₂₈ est un acide gras de C₇ à C₁₇ et n un nombre égal à 2 ou 3, ou parmi des esters glyciniques de la formule générale 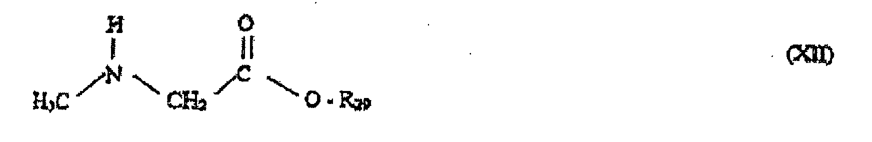 dans laquelle R₂₉ est un groupe alkyle de C₇ à C₁₇, de préférence C₁₂H₂₅.

7. Composition selon les revendications précédentes, **caractérisée en ce que** le régulateur d'humidité est contenu dans une proportion de 50 à 80 % en poids, rapportée à l'ensemble de la composition, et est choisi en particulier parmi des polyols.

8. Composition selon les revendications précédentes, **caractérisée en ce que** le polyol choisi est de la glycérine, du propylène glycol, du butylène glycol, un polyalkylène glycol ou un alcool dérivé d'un sucre.

9. Composition selon les revendications précédentes, **caractérisée en ce qu'**elle contient encore 0,01 à 50 % en poids, de préférence 0,1 à 40 % en poids d'au moins un produit de soins dermatologiques naturel, copie de naturel ou synthétique, de préférence à base naturelle végétale.

10. Composition selon les revendications précédentes, **caractérisée en ce qu'**elle contient encore des substances usuelles auxiliaires et additionnelles, telles des agents de surface non ioniques et/ou cationiques, des charges minérales et/ou organiques, des colorants et/ou des gélifiants naturels ou synthétiques.

11. Composition selon les revendications précédentes, **caractérisée en ce qu'**elle présente un pH de 3 à 8, de préférence de 3,5 à 5.

12. Composition selon les revendications précédentes, **caractérisée en ce qu'**elle présente pour une température d'application de 10 à 90 °C et pour une application par pulvérisation une viscosité de 1 mPas à 700 mPas, de préférence 5 mPas à 600 mPas et, pour une application par rouleau, par racle (tournante) ou par lame une viscosité de 10 mPas à 2000 mPas.

13. Composition selon les revendications précédentes, contenant 40 à 90 % en poids, de préférence 50 à 80 % en poids d'au moins un régulateur d'humidité, 0 à 20 % en poids, de préférence 2 à 10 % en poids d'au moins un agent de surface non ionique et/ou cationique, 1 à 25 % en poids, de préférence 2 à 20 % en poids d'au moins un agent de surface non ionique spécial, choisi parmi des oxydes d'amine et/ou des esters d'hydroxyacides, ainsi que parmi au moins un agent de surface ampholyte, 0,01 à 40 % en poids, de préférence 0,01 à 20 % en poids d'au moins un produit de soins dermatologiques, et 0 à 70 % en poids, de préférence 0 à 40% en poids, d'un gélifiant, le reste étant de l'eau.

14. Procédé de fabrication d'articles en tissu ouate, **caractérisé en ce qu'**on applique une composition selon les revendications 1 à 13 sur le voile de fibres ou sur la nappe de tissu ouate à l'intérieur de la partie filtre, de la partie presse, de la partie TAD, du cylindre frictionneur et/ou de la partie sèche, c.-à-d. pour une densité en produit fibreux de 20 à 97 %, rapporté au poids de fibres sèches de la nappe, dans une quantité de 0,1 à 50 % en poids, de préférence 0,2 à 50 % en poids, continûment ou discontinûment, sur ou dans la nappe, et qu'après l'application, on soumet le cas échéant la nappe à un lissage ultérieur.

15. Procédé de fabrication d'articles en tissu ouate, **caractérisé en ce qu'**on applique une composition selon les revendications 1 à 13 sur le voile de fibres ou sur la nappe de tissu ouate après la partie sèche sur la nappeuse, la bobineuse et/ou sur l'automate de traitement, dans une quantité de 0,1 à 40 % en poids, de préférence de 1 à 20 % en poids, continûment ou discontinûment, sur ou dans la nappe, et qu'après l'application, on soumet le cas échéant la nappe à un lissage ultérieur.

16. Procédé selon la revendication 15, **caractérisé en ce qu'**on applique la composition pour une densité de produit fibreux de 35 à 97 %, rapporté au poids de fibres sèches de la nappe monocouche, dans une quantité de 0,2 à 50 %, de préférence de 1 à 20 %.

17. Procédé selon les revendications 14 à 16, **caractérisé en ce que** la nappe de tissu ouate est une nappe multicouche et **en ce que** la composition est appliquée pour une densité de produit fibreux de plus de 90 %, rapportée au poids des fibres sèches, sur au moins une des couches extérieures de la nappe multicouche dans une quantité de 0,001 à 50 %, de préférence de 0,01 à 30 %.

18. Procédé selon les revendications 17, **caractérisé en ce que** la composition est appliquée sur la nappe multicouche dans une quantité de 0,001 à 50 %, de préférence de 0,01 à 30 %.

19. Procédé selon les revendications 14 à 18, **caractérisé en ce que** l'application d'au moins une partie de la composition sur la nappe s'effectue par pulvérisation, rouleaux, racles (tournantes) ou par le procédé à la lame ou dans et/ou sur la nappe par des microcapsules, insérées dans des micro-éponges ou sous forme de liposomes, en particulier en rapport avec le produit de soins dermatologiques.

20. Procédé selon les revendications 14 à 19, **caractérisé en ce que** le lissage ultérieur, sauf d'après le procédé selon la revendication 19, dernière variante, s'effectue par au moins un passage de la nappe de tissu ouate par une fente d'une paire de rouleaux, pour laquelle un rouleau avec une surface d'acier est associé à un rouleau antagoniste avec une surface d'acier, de plastique, de papier ou de caoutchouc, de préférence une surface de plastique.

21. Procédé selon les revendications 14 à 19, **caractérisé en ce que** le lissage ultérieur, sauf d'après le procédé selon la revendication 19, dernière variante, s'effectue par un double passage de la nappe de tissu ouate par une fente d'une paire de rouleaux pour laquelle d'abord un rouleau avec une surface d'acier est associé à un rouleau antagoniste avec une surface de plastique puis, symétriquement, un rouleau avec une surface de plastique est associé à un rouleau antagoniste avec une surface d'acier.

22. Procédé selon les revendications 14 à 21, **caractérisé en ce que** l'application de la composition sur le voile de fibres s'effectue dans le cadre d'un procédé conventionnel de fabrication de tissu ouate.

23. Procédé selon les revendications 14 à 21, **caractérisé en ce que** l'application de la composition sur le voile de fibres s'effectue dans le cadre d'un séchage par courant traversant (procédé TAD) ou dans le cadre d'un placement de voile de non-tissé.

24. Utilisation de la composition selon les revendications 1 à 13, en particulier sous forme de lotion pour le traitement des produits en tissu ouate, en particulier des mouchoirs de poche en papier, des mouchoirs cosmétiques, des mouchoirs de démaquillage, des mouchoirs de toilette et des serviettes en papier de cuisine, en particulier selon le procédé d'après les revendications 14 à 23.

25. Article en tissu ouate contenant une composition, elle-même contenant un régulateur d'humidité, **caractérisé en ce que** la composition contenant le régulateur d'humidité contient 1 à 25 % en poids, de préférence 2 à 20 % en poids, rapporté à la composition totale, d'au moins un agent de surface spécial non ionique, choisi entre au moins un oxyde d'amine et/ou des esters d'hydroxyacides, à l'exception du lactate de myristyle et des sels de sodium des lactates d'alcools gras, et/ou au moins un agent de surface ampholyte, le régulateur d'humidité étant contenu dans une quantité de 40 à 90 % en poids, rapporté à la composition totale.

26. Article en tissu ouate selon la revendication 25, **caractérisé en ce qu'**il contient 0,05 à 50 % en poids, de préférence 1 à 30 % en poids, rapporté à l'article en tissu ouate, d'un agent de surface spécial non ionique, choisi entre au moins un oxyde d'amine et/ou des esters d'hydroxyacides, à l'exception du lactate de myristyle et des sels de sodium des lactates d'alcools gras, et/ou au moins un agent de surface ampholyte.

27. Article en tissu ouate selon les revendications 25 et 26, **caractérisé en ce qu'**il présente encore sur au moins une partie de sa surface, sur ses faces extérieures, à la face intérieure des couches extérieures, seulement sur les couches intérieures ou sur toutes les couches, une composition selon les revendications 1 à 13.

28. Article en tissu ouate selon les revendications 25 à 27, **caractérisé en ce qu'**au moins une partie de la composition d'après les revendications 1 à 13 est présente sur/dans les couches sous forme d'application, sous forme de microcapsules, insérées dans des micro-éponges ou sous forme de liposomes, de préférence qu'au moins une partie du produit de soins dermatologiques selon la revendication 9 est présente sur/dans les couches sous forme de microcapsules, insérées dans des micro-éponges ou sous forme de liposomes.
